# EUROPÄISCHE PATENTSCHRIFT

(11) **EP 2 231 652 B1**
(45) Veröffentlichungstag und Bekanntmachung des Hinweises auf die Patenterteilung: **30.05.2012**
(21) Anmeldenummer: 08859392.6
(22) Anmeldetag: 02.12.2008
(51) Int. Cl.: C07D 417/04, C07D 417/14, C07D 495/02, A61K 31/427, A61P 1/16, A61P 35/00, A61P 11/00, A61P 17/00, A61P 19/02, A61P 27/02, A61P 37/00

(54) **NEUE 2-HETARYLTHIAZOL-4-CARBONSÄUREAMID-DERIVATE, DEREN HERSTELLUNG UND VERWENDUNG ALS ARZNEIMITTEL**
NOVEL 2-HETARYL THIAZOLE-4-CARBOXAMIDE DERIVATIVES, PRODUCTION THEREOF, AND USE THEREOF AS A MEDICAMENT
NOUVEAUX DÉRIVÉS D'AMIDE D'ACIDE 2-HÉTARYLTHIAZOLE-4-CARBOXYLIQUE, LEUR PRÉPARATION ET LEUR UTILISATION EN TANT QUE MÉDICAMENT

(30) Priorität: 10.12.2007 EP 07076068
(43) Veröffentlichungstag der Anmeldung: 29.09.2010
(73) Patentinhaber: Bayer Pharma Aktiengesellschaft, 13353 Berlin (DE)
(72) Erfinder: BOTHE, Ulrich, 13187 Berlin (DE); VON BONIN, Arne, 16548 Glienicke-Nordbahn (DE); NGUYEN, Duy, 10179 Berlin (DE); BÖMER, Ulf, 16548 Glienicke (DE); GUENTHER, Judith, 13187 Berlin (DE)
(86) Internationale Anmeldenummer: PCT/EP2008/010187
(87) Internationale Veröffentlichungsnummer: WO 2009/074246

(56) Entgegenhaltungen:
- EP-A- 0 928 790
- EP-A- 1 832 586
- WO-A-2004/058751
- WO-A-2005/000793
- WO-A-2007/035478
- WO-A-2008/054702

## Beschreibung

Die vorliegende Erfindung betrifft 2-Hetarylthiazol-4-carbonsäureamid-Derivate der Formel (I), deren Verwendung als Medikament zur Behandlung verschiedener Erkrankungen, sowie Verfahren zur deren Herstellung.

### Biologischer Hintergrund

Für zahlreiche chronisch entzündliche Erkrankungen, wie z.B. Rheumatoider Arthritis, Morbus Crohn, Asthma und Multipler Sklerose, ist ein überreagierendes Immunsystem mit verantwortlich. Durch ein vermehrtes Freisetzen von proinflammatorischen Zytokinen kommt es zu Schädigungen von körpereigenen Gewebestrukturen. Hierbei ist das Zusammenspiel von angeborenem und adaptiven Immunsystem von zentraler Bedeutung (Akira et al., 2001). Eine Modulation des Immunsystems mit Substanzen, die mit der Aktivierung von Zellen des angeborenen und/oder des adaptiven Immunsystem interferieren, wirkt anti-inflammatorisch und kann damit den pathologischen Phänotyp in den oben exemplarisch genannten Erkrankungen abmildern.
Die angeborene Immunität ("innate immunity") beruht darauf, daß Mikroorganismen wie Bakterien und Viren bestimmte inherente Merkmale aufweisen, durch die sie vom Immunsystem erkannt werden und dieses anschließend aktivieren. Erkannt werden bestimmte Pathogen assozierte Muster ("pathogen associated molecular pattern, PAMPs"). PAMPs werden von den "Pattern recognition receptors" (PRR) erkannt, zu denen auch Toll Like Rezeptoren (TLR) gehören (Janeway und Medzhitov, 2002). TLRs sind Homologe zum Drosophila-Rezeptorprotein "toll". Der Mensch hat zehn verschiedene TLRs. TLR eins und sechs sind Corezeptoren für TLR2. TLR2 erkennt u.a. Lipoproteine und Lipopeptide. TLR3 erkennt doppelsträngige RNA. TLR4 erkennt u.a. LPS von gram-negativen und Lipoteichonsäure von gram-positiven Bakterien. TLR5 erkennt Flagellin. TLR9 erkennt CpG-Motive in bakterieller DNA (O'Neill, 2006). Corezeptoren können die Erkennungsfähigkeiten von TLRs weiter verändern (Jiang et al., 2005).

### IL-1/-18, TLR Signaltransduktion

TLRs sind in der Signalübertragung mit IL-1 / IL-18 Zytokinrezeptoren verwandt. IL-1 ("endogenes Pyrogen") stimuliert stark Entzündung und induziert Fieber. Mitglieder der IL-1R/TLR Superfamilie haben eine TIR Domäne (Toll/IL1 Receptor). Die TIR Domäne ist etwa 200 Aminosäuren lang und enthält drei konservierte Sequenzmotive. TIR-Domänen tragende Proteine binden über eine Protein-Protein Interaktion (O'Neill et al., 2005). Die Subklasse eins (IL-1 R Familie) enthält drei Igartige-Domänen, der Rezeptor ist ein Heterodimer. Dazu gehören die IL-1 Rezeptoren eins und zwei, der Co-Rezeptor IL-1RAcP und die entsprechenden Proteine des IL-18 Systems. Die Subklasse zwei (TLR-Familie) enthält Leucin-rich Motife. Toll-like Rezeptoren bilden Homo- oder Heterodimere.

Nach Aktivierung der TLR bzw. IL-1, -18 Rezeptoren durch die entsprechenden Liganden wird eine mehrstufige Signalkaskade in Gang gesetzt. Der TLR bzw. IL-1/- 18 Rezeptorkomplex wechselwirkt über TIR/TIR Kontakte mit dem Adaptorprotein MyD88. Die "IL-1 associated receptor kinase" (IRAK-1) hat normalerweise Tollip (Toll interacting protein) gebunden, das wahrscheinlich als ein abschwächendes Molekül ("silencer") wirkt. IRAK/Tollip bindet an den aktiven TLR/IL-1R Komplex. MyD88 verdrängt Tollip wodurch IRAK1 und IRAK-4 aktiviert wird, höchstwahrscheinlich als Dimer durch Transphosphorylierung. Aktives IRAK verlässt den Rezeptor und bindet im Cytoplasma an das Adaptermolekül TRAF (Barton und Medzhitov, 2003). Über TRAF werden weitere Proteine ubiquitiniliert. Über einen unbekannten Mechanismus führt Ub-TRAF zur Autophosphorylierung der S/T-Kinase TAK1 (eine MAP-Kinase Kinasekinase). TAK1 phosphoryliert IκB (NF-κB Aktivierung) und MKK6. Letztere ist für die Aktivierung der MAP-Kinasen p38 und JNK verantwortlich. NF-κB wurde als Nuclear Factor für die Expression der leichten Antikörperkette Kappa in B-Zellen identifiziert, ist aber an der Regulation vieler anderer Gene ebenfalls beteiligt. NF-κB wird im inaktiven Zustand im Cytoplasma zurückgehalten, wo es an den Inhibitor IκB gebunden ist (Deng et al., 2000). Phosphorylierung von IκB bewirkt, daß der Inhibitor IκB proteolytisch abgebaut wird und der Transkriptionsfaktor in den Kern wandern kann. NF-κB ist ein Heterodimer aus den Untereinheiten p65 (Rel) und p50 (Bäuerle und Henkel, 1994). Es gibt mehrere Mitglieder dieser Familie, die auf unterschiedliche Weise zusammenwirken können. NF-κB alleine kann Transkription nicht auslösen. Für Genaktivierung sind transkriptionelle Coaktivatoren erforderlich, wie etwa p300 oder CBT (Akira und Takeda, 2004).
Nach erfolgter Aktivierung von TIR-Domäne enthaltenden Rezeptoren kommt es unter anderem zur Freisetzung von inflammatorischen Zytokinen, wie z.B. IL-1, IL-6, IL-23 und TNF-alpha (Adachi et al., 1998).

Den strukturell naheliegenden Stand der Technik bilden die Strukturen folgender Patentanmeldungen:

In WO2007/016292 werden N-Aryl-2-arylthiazol-4-carbonsäureamid-Derivate als Biofilmmodulatoren (Modulatoren von Bakterienfilmen) beschrieben, die aufgrund des bicyclischen C-D-Ringsystemes unterschiedlich von den hier beanspruchten Verbindungen sind.
In W02007/035478 werden Verbindungen offenbart, die anstelle einer Carboxamidgruppe eine Carboxylgruppe in ortho-Positon aufweisen.
In US 6,274,738 werden N-Aryl-2-pyridylthiazol-4-carbonsäureamid-Derivate als Verbindungen beschrieben, die die DNA Primase modulieren. Allerdings weisen diese Verbindungen an der N-Arylgruppe keine Aminocarbonylgruppe auf, die in ortho-Postion bezüglich der Pyridylthiazol-4-carbonsäureamideinheit steht.
Darüber hinaus werden in US 4,879295 N-Tetrazolylthiazolcarboxamide offenbart. Thiazolamidderivate werden in WO2006/122011 als Inhibitoren der Virusreplikation genannt. In WO2005/048953 werden Thiazolamidderivate verknüpft mit einer Isoxazoleinheit als Kinaseinhibitoren berschrieben. Die Strukturen unterscheiden sich allerdings von den Strukturen der vorliegenden Erfindung.
Auch die in der WO2007/052882 offenbarten Verbindungen weisen keine Aminocarbonylgruppe in ortho-Position zur Aminocarbonylthiazolgruppe auf. Besonderes Merkmal der in WO2004072025 beschriebenen Verbindungen ist im Gegensatz zu den hier offenbarten Strukturen ein Pyrrolidinring, der zusätzlich noch mit einem weiteren Substituenten (wie z. B. einer Dimethylaminogruppe) über ein Stickstoffatom verknüpft vorliegt.

In W0200512256, W0200677424, WO2006077425 und W0200677428 werden Pyrazolderivate als Kinaseinhibitoren offenbart. Unter anderem sind auch Strukturen beschrieben, bei denen ein Thiazolamid mit der Pyrazoleinheit verknüpft ist, wobei allerdings keines der Pyrazol-Stickstoffatome methyliert vorliegt und die Pyrazoleinheit auch nicht mit einer Aminocarbonylgruppe (C(O)NH₂) substituiert ist.

Pyrazolamide, die aber gleichzeitig mit einer Harnstoffeinheit verknüpft vorliegen, werden in W02005/37797 beschrieben.

In W02005/115986 werden Pyridinamide beansprucht, wobei aber keine Verknüpfungen mit schwefelhaltigen Heterocyclen wie Thiazol vorgesehen sind.

Strukturell unterschiedlich zu den hier beschriebenen Verbindungen sind auch die in WO2005/049604 beschriebenen Pyridinamide, aufgrund der Verknüpfung mit einem sauerstoffsubstituierten Phenylring.

In EP1666455 werden Amide mit zusätzlicher Carboxamidstruktur beschrieben, wobei der Substituent R1 keine Aminocarbonylgruppe sein kann.

Ausgehend von diesem Stand der Technik besteht die Aufgabe der vorliegenden Erfindung, weitere Strukturen für die Therapie bereitzustellen, insbesondere für die Immunmodulation.

Die Aufgabe wird gelöst durch Verbindungen der allgemeinen Formel (I), mit den Bausteinen A, B, C und D , 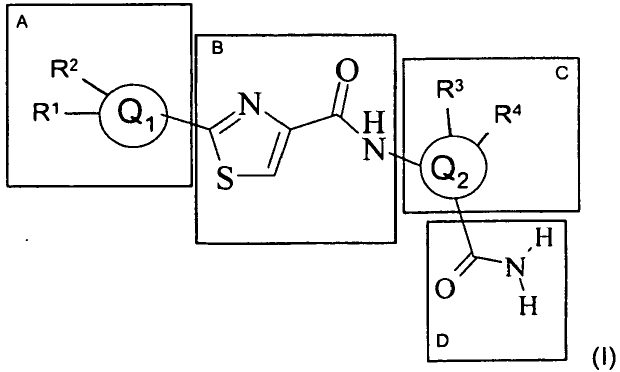 in der
die Bausteine B und D in ortho-Stellung zueinander stehen und
- Q₁: für einen Heteroarylring mit 6 Ringatomen steht, und
- R¹ und R²: unabhängig voneinander stehen für
(i) Wasserstoff, Hydroxy, Nitro, Halogen, Cyano, -CF₃, -NR⁵R⁶ oder
(ii) -C(O)-OR¹⁰, -C(O)-R⁷, -C(O)-C₁-C₃-Fluoralkyl, -C(O)-NR⁵R⁶, -NH-C(O)-R⁷ oder
(iii) einen C₁-C₆-Alkyl-, C₂-C₆ -Alkenyl-, C₂-C₆-Alkinyl-, C₁-C₆-Alkoxy-, C₁-C₆-Fluoralkyl- oder einen C₁-C₆-Fluoralkoxyrest,
   jeweils gegebenenfalls mit -C₁-C₃-Alkoxy, Hydroxy, -C(O)-OR¹⁰ oder -NR⁸R⁹ ein- oder mehrfach, gleich oder verschieden substituiert, oder
(iv) -O-SO₂-NR⁵R⁶, -SO₂-R⁷, -SO₂-NR⁵R⁶, und
- Q₂: für einen Aryl-, Heteroaryl- oder einen hydrierten bizyklischen Heteroarylring steht, und
- R³ und R⁴: unabhängig voneinander stehen für
(i) Wasserstoff, Halogen oder-NR¹¹R¹², oder
(ii) einen C₁-C₆-Alkyl- oder C₁-C₆-Alkoxyrest, die jeweils gegebenenfalls ein- oder mehrfach, gleich oder verschieden mit Hydroxy, C₁-C₃-Alkoxy, -NR⁸R³ oder Heterocyclyl substituiert sind, wobei der Heterocylylring ein- oder mehrfach, gleich oder verschieden substituiert sein kann mit C₁-C₃-Alkyl oder -C(O)-R⁷ , wobei
- R⁵ und R⁶: unabhängig voneinander stehen für Wasserstoff oder einen C₁-C₆ Alkylrest, der gegebenenfalls ein- oder mehrfach, gleich oder verschieden mit Hydroxy, -C₁-C₃-Alkoxy, -NR⁸R⁹ oder Heterocyclyl substituiert ist, wobei der Heterocylylring ein- oder mehrfach, gleich oder verschieden substituiert sein kann mit C₁-C₃-Alkyl oder -C(O)-R⁷, oder
- R⁵ und R⁶: bilden alternativ gemeinsam mit dem Stickstoffatom einen 5- bis 7-gliedrigen Ring, der gegebenenfalls zusätzlich zum Stickstoffatom ein weiteres Heteroatom enthält und der gegebenenfalls mit C₁-C₆-Alkyl und/oder mit -C(O)-R⁷ ein- oder mehrfach, gleich oder verschieden substituiert ist, und
- R⁷: für einen C₁-C₆ Alkylrest steht, und
- R⁸, R⁹, R¹⁰: unabhängig voneinander stehen für Wasserstoff oder einen C₁-C₆ Alkylrest, und
- R¹¹ und R¹²: unabhängig voneinander stehen für Wasserstoff oder einen C₁-C₆ Alkylrest, der gegebenenfalls ein- oder mehrfach, gleich oder verschieden mit Hydroxy, -C₁-C₃-Alkoxy, -NR⁸R⁹ oder Heterocyclyl substituiert ist, wobei der Heterocylylring ein- oder mehrfach, gleich oder verschieden substituiert sein kann mit C₁-C₃-Alkyl oder -C(O)-R⁷,
sowie deren Salze, Enantiomere und Diastereomere.

Der Erfindung liegen folgende Definitionen zu Grunde:

### Cₙ-Alkyl:

Monovalenter, geradkettiger oder verzweigter, gesättigter Kohlenwasserstoffrest mit n Kohlenstoffatomen.

Ein C₁-C₆ Alkylrest umfasst unter anderem beispielsweise:
Methyl-, Ethyl-, Propyl-, Butyl-, Pentyl-,Hexyl-, *iso*-Propyl-, *iso*-Butyl-, *sec*-Butyl, *tert-*Butyl-, *iso*-Pentyl-, 2-Methylbutyl-, 1-Methylbutyl-, 1-Ethylpropyl-, 1,2-Dimethylpropyl, *neo*-Pentyl-, 1,1-Dimethylpropyl-, 4-Methylpentyl-, 3-Methylpentyl-, 2-Methylpentyl-, 1-Methylpentyl-, 2-Ethylbutyl-, 1-Ethylbutyl-, 3,3-Dimethylbutyl-, 2,2-Dimethylbutyl-, 1,1-Dimethylbutyl-, 2,3-Dimethylbutyl-, 1,3-Dimethylbutyl- 1,2-Dimethylbutyl-.
Bevorzugt ist ein Methyl-, Ethyl-, Propyl- oder Isopropylrest.

### Cₙ-Fluoralkyl:

Monovalenter, geradkettiger oder verzweigter, gesättigter, vollständig oder teilweise fluorierter Kohlenwasserstoffrest mit n Kohlenstoffatomen.

Ein C₁-C₆ Fluoralkylrest umfasst unter anderem beispielsweise:
Trifluormethyl, Difluormethyl, Monofluormethyl, Pentafluorethyl, Heptafluorpropyl, 2,2,2-Trifluorethyl, 3,3,3-Trifluorpropyl, 5,5,6,6,6-Pentafluorhexyl, Pentafluorallyl, 1,1,1,3,3,3-Hexafluor-2-propyl
Bevorzugt ist ein Trifluormethyl, 2,2,2-Trifluorethyl und Pentafluorethylrest.

### Cₙ-Alkenyl:

monovalenter, geradkettiger oder verzweigter Kohlenwasserstoffrest mit n Kohlenstoffatomen und mindestens einer Doppelbindung.

Ein C₂-C₆ Alkenylrest umfasst unter anderem beispielsweise:
Vinyl-, Allyl-, (E)-2-Methylvinyl-, (Z)-2-Methylvinyl-, Homoallyl-, (E)-But-2-enyl-, (Z)-But-2-enyl-, (E)-But-1-enyl-, (Z)-But-1-enyl-, Pent-4-enyl-, (E)-Pent-3-enyl-, (Z)-Pent-3-enyl-, (E)-Pent-2-enyl-, (Z)-Pent-2-enyl-, (E)-Pent-1-enyl-, (Z)-Pent-1-enyl-, Hex-5-enyl-, (E)-Hex-4-enyl-, (Z)-Hex-4-enyl-, (E)-Hex-3-enyl-, (Z)-Hex-3-enyl-, (E)-Hex-2-enyl-, (Z)-Hex-2-enyl-, (E)-Hex-1-enyl-, (Z)-Hex-1-enyl-, Isopropenyl-, 2-Methylprop-2-enyl-, 1-Methylprop-2-enyl-, 2-Methylprop-1-enyl-, (E)-1-Methylprop-1-enyl-, (Z)-1-Methylprop-1-enyl-, 3-Methylbut-3-enyl-, 2-Methylbut-3-enyl-, 1-Methylbut-3-enyl-, 3-Methylbut-2-enyl-, (E)-2-Methylbut-2-enyl-, (Z)-2-Methylbut-2-enyl-, (E)-1-Methylbut-2-enyl-, (Z)-1-Methylbut-2-enyl-, (E)-3-Methylbut-1-enyl-, (Z)-3-Methylbut-1-enyl-, (E)-2-Methylbut-1-enyl-,
(Z)-2-Methylbut-1-enyl-, (E)-1-Methylbut-1-enyl-, (Z)-1-Methylbut-1-enyl-, 1,1-Dimethylprop-2-enyl-, 1-Ethylprop-1-enyl-, 1-Propylvinyl-, 1-Isopropylvinyl-, 4-Methylpent-4-enyl-, 3-Methylpent-4-enyl-, 2-Methylpent-4-enyl-, 1-Methylpent-4-enyl-, 4-Methylpent-3-enyl-, (E)-3-Methylpent-3-enyl-, (Z)-3-Methylpent-3-enyl-, (E)-2-Methylpent-3-enyl-, (Z)-2-Methylpent-3-enyl-, (E)-1-Methylpent-3-enyl-, (Z)-1-Methylpent-3-enyl-, (E)-4-Methylpent-2-enyl-, (Z)-4-Methylpent-2-enyl-, (E)-3-Methylpent-2-enyl-, (Z)-3-Methylpent-2-enyl-, (E)-2-Methylpent-2-enyl-, (Z)-2-Methylpent-2-enyl-, (E)-1-Methylpent-2-enyl-, (Z)-1-Methylpent-2-enyl-, (E)-4-Methylpent-1-enyl-, (Z)-4-Methylpent-1-enyl-, (E)-3-Methylpent-1-enyl-, (Z)-3-Methylpent-1-enyl-, (E)-2-Methylpent-1-enyl-, (Z)-2-Methylpent-1-enyl-, (E)-1-Methylpent-1-enyl-, (Z)-1-Methylpent-1-enyl-, 3-Ethylbut-3-enyl-, 2-Ethylbut-3-enyl-, 1-Ethylbut-3-enyl-, (E)-3-Ethylbut-2-enyl-, (Z)-3-Ethylbut-2-enyl-, (E)-2-Ethylbut-2-enyl-, (Z)-2-Ethylbut-2-enyl-, (E)-1-Ethylbut-2-enyl-, (Z)-1-Ethylbut-2-enyl-, (E)-3-Ethylbut-1-enyl-, (Z)-3-Ethylbut-1-enyl-, 2-Ethylbut-1-enyl-, (E)-1-Ethylbut-1-enyl-, (Z)-1-Ethylbut-1-enyl-, 2-Propylprop-2-enyl-, 1-Propylprop-2-enyl-, 2-Isopropylprop-2-enyl-, 1-Isopropylprop-2-enyl-, (E)-2-Propylprop-1-enyl-, (Z)-2-Propylprop-1-enyl-, (E)-1-Propylprop-1-enyl-, (Z)-1-Propylprop-1-enyl-, (E)-2-Isopropylprop-1-enyl-, (Z)-2-Isopropylprop-1-enyl-, (E)-1-Isopropylprop-1-enyl-, (Z)-1-Isopropylprop-1-enyl-,
(E)-3,3-Dimethylprop-1-enyl-, (Z)-3,3-Dimethylprop-1-enyl-, 1-(1,1-Dimethylethyl)ethenyl.

Bevorzugt ist ein Vinyl- oder Allylrest.

### Cₙ-Alkinyl:

Monovalenter, geradkettiger oder verzweigter Kohlenwasserstoffrest mit n Kohlenstoffatomen und mindestens einer Dreifachbindung.

Ein C₂-C₆ Alkinylrest umfasst unter anderem beispielsweise:
Ethinyl-, Prop-1-inyl-, Prop-2-inyl-, But-1-inyl-, But-2-inyl-, But-3-inyl-, Pent-1-inyl-, Pent-2-inyl-, Pent-3-inyl-, Pent-4-inyl-, Hex-1-inyl-, Hex-2-inyl-, Hex-3-inyl-, Hex-4-inyl-, Hex-5-inyl-, 1-Methylprop-2-inyl-, 2-Methylbut-3-inyl-, 1-Methylbut-3-inyl-, 1-Methylbut-2-inyl-, 3-Methylbut-1-inyl-, 1-Ethylprop-2-inyl-, 3-Methylpent-4-inyl, 2-Methylpent-4-inyl, 1-Methylpent-4-inyl, 2-Methylpent-3-inyl, 1-Methylpent-3-inyl, 4-Methylpent-2-inyl, 1-Methylpent-2-inyl, 4-Methylpent-1-inyl, 3-Methylpent-1-inyl, 2-Ethylbut-3-inyl-, 1-Ethylbut-3-inyl-, 1-Ethylbut-2-inyl-, 1-Propylprop-2-inyl-, 1-Isopropylprop-2-inyl-, 2,2-Dimethylbut-3-inyl-, 1,1-Dimethylbut-3-inyl-, 1,1-Dimethylbut-2-inyl- oder eine 3,3-Dimethylbut-1-inyl-.

Bevorzugt ist ein Ethinyl-, Prop-1-inyl- oder Prop-2-inylrest.

### Cₙ-Cycloalkyl:

Monovalenter, cyclischer Kohlenwasserstoffring mit n Kohlenstoffatomen.

C₃-C₇-Cyclolalkylring umfasst:
Cyclopropyl, Cyclobutyl, Cyclopentyl, Cyclohexyl- und Cycloheptyl.

Bevorzugt ist ein Cyclopropyl-, Cyclobutyl-, Cylopentyl- oder ein Cyclohexylring.

### Cₙ₋Alkoxy:

Geradkettiger oder verzweigter Cₙ-Alkyletherrest der Formel -OR mit R=Alkyl.

### Cₙ-Fluoralkoxy:

Geradkettiger oder verzweigter Cₙ-Fluoralkyletherrest der Formel -OR mit R=Cₙ-Fluoralkyl.

### Cₙ-Aryl

Cₙ-Aryl ist ein monovalentes, aromatisches Ringsystem ohne Heteroatom mit n Kohlenwasserstoffatomen.
C₆-Aryl ist gleich Phenyl. C₁₀-Aryl ist gleich Naphthyl.
Bevorzugt ist Phenyl.

### Heteroatome

Unter Heteroatomen sind Sauerstoff-, Stickstoff- oder Schwefel-Atome zu verstehen.

### Heteroaryl

Heteroaryl ist ein monovalentes, aromatisches Ringsystem mit mindestens einem von einem Kohlenstoff verschiedenen Heteroatom. Als Heteroatome können Stickstoffatome, Sauerstoffatome und/oder Schwefelatome vorkommen. Die Bindungsvalenz kann an einem beliebigen aromatischen Kohlenstoffatom oder an einem Stickstoffatom sein.

Ein monocyclischer Heteroarylring gemäß der vorliegenden Erfindung hat 5 oder 6 Ringatome.

Heteroarylringe mit 5 Ringatomen umfassen beispielsweise die Ringe:
Thienyl, Thiazolyl, Furanyl, Pyrrolyl, Oxazolyl, Imidazolyl, Pyrazolyl, Isoxazolyl, Isothiazolyl, Oxadiazolyl, Triazolyl, Tetrazolyl und Thiadiazolyl.

Heteroarylringe mit 6 Ringatomen umfassen beispielsweise die Ringe:
Pyridinyl, Pyridazinyl, Pyrimidinyl, Pyrazinyl und Triazinyl.

Ein bicyclischer Heteroarylring gemäß der vorliegenden Erfindung hat 9 bis 10 Ringatome.

Heteroarylringe mit 9 Ringatomen umfassen beispielsweise die Ringe:
Indolyl, Isoindolyl, Indazolyl, Benzothiazolyl, Benzofuranyl, Benzothiophenyl, Benzimidazolyl, Benzoxazolyl, Azocinyl, Indolizinyl, Purinyl.

Heteroarylringe mit 10 Ringatomen umfassen beispielsweise die Ringe:
Isochinolinyl, Chinolinyl, Cinnolinyl, Chinazolinyl, Chinoxalinyl, Phthalazinyl, 1,7- oder 1,8-Naphthyridinyl, Pteridinyl

Monocyclische Heteroarylringe mit 5 oder 6 Ringatomen sind bevorzugt.

Hydrierte bicyclische Aryl- oder Heteroarylringe sind Bizyklen, bei denen ein Ring teilweise oder vollständig hydriert vorliegt. Die Bindungsvalenz kann an einem beliebigen Atom des aromatischen Teiles des hydrierten bicyclischen Aryl- oder Heteroarylringes sein.

Hydrierte bicyclische Aryl- oder Heteroarylringe umfassen beispielsweise die Ringe: Indanyl, 1,2,3,4-Tetrahydro-naphthalenyl, 1,2,3,4-Tetrahydro-isochinolinyl, 1,2,3,4-Tetrahydro-chinolinyl, 2,3-Dihydro-1H-indolyl, 2,3-Dihydro-1H-isoindolyl, 4,5,6,7-Tetrahydro-thieno[2,3-c]pyridinyl, 4,5,6,7-Tetrahydro-thieno[3,2-c]pyridinyl, 2,3-Dihydro-benzofuranyl, Benzo[1,3]dioxolyl, 2,3-Dihydro-benzo[1,4]dioxinyl, Chromanyl, 1,2,3,4-Tetrahydro-chinoxalinyl, 3,4-Dihydro-2H-benzo[1,4]oxazinyl.

Hydrierte bicyclische Aryl- oder Heteroarylringe bei denen ein Ring teilweise oder vollständig hydriert vorliegt, können gegebenenfalls eine oder zwei Carbonylgruppen im Ringsystem enthalten.
Beispiele hierfür sind Indolonyl, Isoindolonyl, Benzoxazinonyl, Phthalazinonyl, Chinolonyl, Isochinolonyl, Phthalidyl, Thiophthalidyl.

### Heterocyclyl

Heterocyclyl im Sinne der Erfindung ist ein vollständig hydriertes Heteroaryl (vollständig hydriertes Heteroaryl = gesättigtes Heterocyclyl) d.h. ein nicht aromatisches Ringsystem mit mindestens einem von einem Kohlenstoff verschiedenen Heteroatom. Als Heteroatome können Stickstoffatome, Sauerstoffatome und/oder Schwefelatome vorkommen. Die Bindungsvalenz kann an einem beliebigen Kohlenstoffatom oder an einem Stickstoffatom sein.

Heterocycylring mit 3 Ringatomen umfasst beispielsweise:
Aziridinyl.

Heterocycylring mit 4 Ringatomen umfasst beispielsweise:
Azetidinyl, Oxetanyl.

Heterocycylringe mit 5 Ringatomen umfassen beispielsweise die Ringe:
Pyrrolidinyl, Imidazolidinyl, Pyrazolidinyl und Tetrahydrofuranyl.

Heterocyclylringe mit 6 Ringatomen umfassen beispielsweise die Ringe:
Piperidinyl, Piperazinyl, Morpholinyl, Tetrahydropyranyl und Thiomorpholinyl

Heterocycylringe mit 7 Ringatomen umfassen beispielsweise:
Azepanyl, Oxepanyl, [1,3]-Diazepanyl, [1,4]-Diazepanyl.

Heterocycylringe mit 8 Ringatomen umfassen beispielsweise:
Oxocanyl, Azocanyl.

Heterocyclylringe können gegebenenfalls teilweise ungesättigt sein und/oder auch eine Carbonylgruppe im Ring enthalten.
Beispiele hierfür sind Dihydro-furan-2-onyl, Pyrrolidin-2-onyl, Piperazin-2-onyl, Morpholin-2-onyl, 3(2H)-Pyridazinonyl, 5,6-Dihydro-2-pyran-2-onyl, 5,6-Dihydropyridin-2(1H)-onyl, 2-Piperidonyl, 1,2,3,6-Tetrahydropyridinyl.

### Halogen

Die Bezeichnung Halogen umfasst Fluor, Chlor, Brom und lod.

Ebenfalls als von der vorliegenden Erfindung als erfasst anzusehen sind alle Verbindungen, die sich ergeben durch jede mögliche Kombination der oben genannten möglichen, bevorzugten und besonders bevorzugten Bedeutungen der Substituenten.

Besondere Ausführungsformen der Erfindung bestehen darüber hinaus in Verbindungen, die sich durch Kombination der direkt in den Beispielen offenbarten Bedeutungen für die Substituenten ergeben.

Ebenfalls als von der vorliegenden Erfindung als erfasst anzusehen sind die Salze der Verbindungen.

Die Formulierung der erfindungsgemäßen Verbindungen zu pharmazeutischen Präparaten erfolgt in an sich bekannter Weise, indem man den oder die Wirkstoffe mit den in der Galenik gebräuchlichen Hilfsstoffen in die gewünschte Applikationsform überführt.

Als Hilfsstoffe können dabei beispielsweise Trägersubstanzen, Füllstoffe, Sprengmittel, Bindemittel, Feuchthaltemittel, Gleitmittel, Ab- und Adsorptionsmittel, Verdünnungsmittel, Lösungsmittel, Cosolventien, Emulgatoren, Lösungsvermittler, Geschmackskorrigentien, Färbemittel, Konservierungs-, Stabilisierungs-, Netzmittel, Salze zur Veränderung des osmotischen Drucks oder Puffer zum Einsatz kommen. Dabei ist auf Remington's Pharmaceutical Science, 15th ed. Mack Publishing Company, East Pennsylvania (1980) hinzuweisen.

Die pharmazeutischen Formulierungen können
in fester Form, zum Beispiel als Tabletten, Dragees, Pillen, Suppositorien, Kapseln, transdermale Systeme oder
in halbfester Form, zum Beispiel als Salben, Cremes, Gele, Suppositiorien, Emulsionen oder
in flüssiger Form, zum Beispiel als Lösungen, Tinkturen, Suspensionen oder Emulsionen vorliegen.

Hilfsstoffe im Sinne der Erfindung können beispielsweise Salze, Saccharide (Mono-, Di-, Tri-, Oligo-, und/oder Polysaccharide), Proteine, Aminosäuren, Peptide, Fette, Wachse, Öle, Kohlenwasserstoffe sowie deren Derivate sein, wobei die Hilfsstoffe natürlichen Ursprungs sein können oder synthetisch bzw. partial synthetisch gewonnen werden können.

Für die orale oder perorale Applikation kommen insbesondere Tabletten, Dragees, Kapseln, Pillen, Pulver, Granulate, Pastillen, Suspensionen, Emulsionen oder Lösungen in Frage.
Für die parenterale Applikation kommen insbesondere Suspensionen, Emulsionen und vor allem Lösungen in Frage.

Aufgrund ihrer anti-entzündlichen und zusätzlichen immunsuppressiven Wirkung können die erfindungsgemäßen Verbindungen der allgemeinen Formel (I) als Medikamente zur Behandlung oder Prophylaxe folgender Krankheitszustände bei Säugetieren und Menschen, für die lokale und systemische Applikation Verwendung finden:
(i) Lungenerkrankungen, die mit entzündlichen, allergischen und / oder proliferativen Prozessen einhergehen:
   - Chronisch obstruktive Lungenerkrankungen jeglicher Genese, vor allem Asthma bronchiale
   - Bronchitis unterschiedlicher Genese
   - Adult respiratory distress syndrome (ARDS), akutes Atemnotssyndrom
   - Bronchiektasen
   - Alle Formen der restriktiven Lungenerkrankungen, vor allem allergische Alveolitis,
   - Lungenödem, insbesondere allergisches
   - Sarkoidosen und Granulomatosen, insbesondere Morbus Boeck
(ii) Rheumatische Erkrankungen / Autoimmunerkrankungen / Gelenkerkrankungen, die mit entzündlichen, allergischen und / oder proliferativen Prozessen einhergehen:
   - Alle Formen rheumatischer Erkrankungen, insbesondere rheumatoide Arthritis, akutes rheumatisches Fieber, Polymyalgia rheumatica, Morbus Behcet
   - Reaktive Arthritis
   - Entzündliche Weichteilerkrankungen sonstiger Genese
   - Arthritische Symtome bei degenerativen Gelenkerkrankungen (Arthrosen)
   - Vitiligo
   - Kollagenosen jeglicher Genese, z.B. systemischer Lupus erythematodes, Sklerodermie, Polymyositis, Dermatomyositis- Sjögren-Syndrom, Still-Syndrom, Felty-Syndrom
   - Sarkoidosen und Granulomatosen
   - Weichteilrheumatismus
(iii) Allergien oder pseudoallergische Erkrankungen, die mit entzündlichen, und / oder proliferativen Prozessen einhergehen:
   - alle Formen allergischer Reaktionen, z.B. Quincke Ödem, Heuschnupfen, Insektenstich, allergische Reaktionen auf Arzneimittel, Blutderivate, Kontrastmittel etc., Anaphylaktischer Schock, Urtikaria, allergische und irritative Kontakdermatitis, allergische Gefäßerkrankungen
   - Vasculitis allergica
(iv) Gefäßentzündungen (Vaskulitiden)
   - Panarteriitis nodosa, Arteriitis temporalis, Erythema nodosum
   - Polyarteritis nodosa
   - Wegner Granulomatose
   - Riesenzellarteriitis
(v) Dermatologische Erkrankungen, die mit entzündlichen, allergischen und / oder proliferativen Prozessen einhergehen:
   - Atopische Dermatitis (vor allem bei Kindern)
   - Sämtliche Ekzemformen wie z.B. atopisches Ekzem (v.a. bei Kindern)
   - Exantheme jedweder Genese oder Dermatosen
   - Psoriasis und Parapsoriasis-Formenkreis
   - Pityriasis rubra pilaris
   - Erythematöse Erkrankungen, ausgelöst durch unterschiedlichen Noxen, z.B. Strahlen, Chemikalien, Verbrennungen etc.
   - Bullöse Dermatosen wie z.B. autoimmuner Pemphigus vulgaris, bullöses Pemphigoid
   - Erkrankungen des lichenoiden Formenkreises,
   - Pruritus (z. B. allergischer Genese)
   - Rosacea-Formenkreis
   - Erythema exsudativum multiforme
   - Manifestation von Gefäßerkrankungen
   - Haarausfall wie Alopecia areata
   - Kutane Lymphome
(vi) Nierenerkrankungen, die mit entzündlichen, allergischen und / oder proliferativen Prozessen einhergehen:
   - Nephrotisches Syndrom
   - Alle Nephritiden, z.B. Glomerulonephritis
(vii) Lebererkrankungen, die mit entzündlichen, allergischen und / oder proliferativen Prozessen einhergehen:
   - akute Hepatitis unterschiedlicher Genese
   - chronisch aggressive und / oder chronisch intermittierende Hepatitis
(viii) Gastrointestinale Erkrankungen, die mit entzündlichen, allergischen und / oder proliferativen Prozessen einhergehen:
   - regionale Enteritis (Morbus Crohn)
   - Colitis Ulcerosa
   - Gastroenteritiden anderer Genese, z.B. einheimische Sprue
(ix) Augenerkrankungen, die mit entzündlichen, allergischen und / oder proliferativen Prozessen einhergehen:
   - allergische Keratitis, Uveitis, Iritis,
   - Konjunktivitis
   - Blepharitis
   - Neuritis nervi optici
   - Chorioiditis
   - Ophthalmia sympathica
(x) Erkrankungen des Hals-Nasen-Ohren-Bereiches, die mit entzündlichen, allergischen und / oder proliferativen Prozessen einhergehen:
   - allergische Rhinitis, Heuschnupfen
   - Otitis externa, z.B. bedingt durch Kontaktekzem
(xi) Neurologische Erkrankungen, die mit entzündlichen, allergischen und / oder proliferativen Prozessen einhergehen:
   - Hirnödem, vor allem allergisches Hirnödem
   - Multiple Sklerose
   - akute Encephalomyelitis
   - Meningitis, vor allem allergische
   - Guillain-Barre Syndrom
   - Morbus Alzheimer
(xii) Bluterkrankungen, die mit entzündlichen, allergischen und / oder proliferativen Prozessen einhergehen wie z. B.: M. Hodgkin oder Non-Hodgkin Lymphome, Thrombozytaemien, Erythrozytosen
   - Erworbene hämolytische Anämie
   - Idiopathische Thrombozytopenie
   - Idiopathische Granulozytopenie
(xiii) Tumorerkrankungen, die mit entzündlichen, allergischen und / oder proliferativen Prozessen einhergehen
   - Akute lymphatische Leukämie
   - Maligne Lymphome
   - Lymphogranulomatosen
   - Lymphosarkome
(xiv) Endokrine Erkrankungen, die mit entzündlichen, allergischen und / oder proliferativen Prozessen einhergehen wie z. B.:
   - Endokrine Orbitopathie
   - Thyreoiditis de Quervain
   - Hashimoto Thyreoiditis
   - Morbus Basedow
   - Granulomatous thyroiditis
   - Struma lymphomatosa
   - Autoimmune adrenalitis
   - Diabetes mellitus, insbesondere Typ 1 Diabetes
   - Endometriose
(xv) Organ- und Gewebstransplantationen, Graft-versus-host-disease
(xvi) Schwere Schockzustände, z.B anaphylaktischer Schock, systemic inflammatory response syndrome (SIRS)

Ein Gegenstand der Erfindung ist die Verwendung der erfindungsgemäßen Verbindungen der allgemeinen Formel (I) zur Herstellung eines Arzneimittels.

Ein weiterer Gegenstand der Erfindung ist die Verwendung der erfindungsgemäßen Verbindungen zur Behandlung von Erkrankungen, die mit entzündlichen, allergischen und / oder proliferativen Prozessen einhergehen.
In der allgemeinen Formel (I) kann Q₁ stehen für einen Heteroarylring mit 6 Ringatomen.

Bevorzugt steht Q₁ für einen Pyridinyl-, Pyridazinyl-, Pyrimidinyl-, Pyrazinyl- oder Triazinylring.

Besonders bevorzugt steht Q₁ für einen Pyridyl- oder Pyrazinylring.

In der allgemeinen Formel (I) können R¹ und R² unabhängig voneinander stehen für:
(i) Wasserstoff, Hydroxy, Nitro, Halogen, Cyano, -CF₃, -NR⁵R⁶ oder
(ii) -C(O)-OR¹⁰, -C(O)-R⁷, -C(O)-C₁-C₃-Fluoralkyl, -C(O)-NR⁵R⁶, -NH-C(O)-R⁷ oder
(iii) einen C₁-C₆-Alkyl-, C₂-C₆ -Alkenyl-, C₂-C₆-Alkinyl-, C₁-C₆-Alkoxy-, C₁-C₆-Fluoralkyl- oder einen C₁-C₆-Fluoralkoxyrest,
   jeweils gegebenenfalls mit -C₁-C₃-Alkoxy, Hydroxy, -C(O)-OR¹⁰ oder -NR⁸R⁹ ein- oder mehrfach, gleich oder verschieden substituiert, oder
(iv) -O-SO₂-NR⁵R⁶, -SO₂-R⁷, -SO₂-NR⁵R⁶.

Bevorzugt stehen R¹ und R² unabhängig voneinander für:
(i) Wasserstoff, Hydroxy, Halogen, Cyano, -CF₃, -NR⁵R⁶ oder
(ii) einen C₁-C₆-Alkyl-, C₁-C₆-Alkoxy-, C₁-C₆-Fluoralkyl- oder einen C₁-C₆-Fluoralkoxyrest oder
(iii) -SO₂-R⁷.

Besonders bevorzugt stehen R¹ und R² unabhängig voneinander für:
(i) Wasserstoff, -NH₂, Hydroxy, Halogen, -CF₃, oder
(ii) einen C₁-C₆-Alkoxy-, C₁-C₆-Fluoralkyl- oder einen C₁-C₆-Fluoralkoxyrest.

In der allgemeinen Formel (I) kann Q₂ stehen für einen Aryl-, Heteroaryl- oder einen hydrierten bizyklischen Heteroarylring.

Bevorzugt steht Q₂ für einen:
Phenyl-, Thienyl-, Thiazolyl-, Furanyl-, Pyrrolyl-, Oxazolyl-, Imidazolyl-, Pyrazolyl-, Isoxazolyl, Isothiazolyl, 1,2,3-Triazolyl, Pyridinyl-, Pyridazinyl-, Pyrimidinyl-, Pyrazinyl-, 1,2,3,4-Tetrahydro-isochinolinyl-, 1,2,3,4-Tetrahydro-chinolinyl-, 2,3-Dihydro-1H-indolyl-, 2,3-Dihydro-1H-isoindolyl-, 4,5,6,7-Tetrahydro-thieno[2,3-c]pyridinyl-, 4,5,6,7-Tetrahydro-thieno[3,2-c]pyridinyl-, 2,3-Dihydro-benzofuranyl-, Benzo[1,3]dioxolyl-, 2,3-Dihydro-benzo[1,4]dioxinyl-, 1,2,3,4-Tetrahydro-chinoxalinyl- oder einen 3,4-Dihydro-2H-benzo[1,4]oxazinylring.

Besonders bevorzugt steht Q₂ für einen Phenyl-, Pyrazolyl-, Thienyl-, Pyridinyl- oder 4,5,6,7-Tetrahydro-thieno[2,3-c]pyridinylring.

In der allgemeinen Formel (I) können R³ und R⁴ unabhängig voneinander stehen für:
(i) Wasserstoff, Halogen oder -NR¹¹R¹², oder
(ii) einen C₁-C₆-Alkyl- oder C₁-C₆-Alkoxyrest, die jeweils gegebenenfalls ein- oder mehrfach, gleich oder verschieden mit Hydroxy, C₁-C₃-Alkoxy, -NR⁸R⁹ oder Heterocyclyl substituiert sind, wobei der Heterocylylring ein- oder mehrfach, gleich oder verschieden substituiert sein kann mit C₁-C₃-Alkyl oder -C(O)-R⁷.

Bevorzugt stehen R³ und R⁴ unabhängig voneinander für:
(i) Wasserstoff, Halogen, -NR¹¹R¹² oder
(ii) einen C₁-C₆-Alkyl- oder C₁-C₆-Alkoxyrest,
jeweils gegebenenfalls mit Morpholin oder -NR⁸R⁹ substituiert.

Besonders bevorzugt stehen R³ und R⁴ unabhängig voneinander für Wasserstoff, Halogen oder C₁-C₆-Alkyl.

In der allgemeinen Formel (I) können R⁵ und R⁶ unabhängig voneinander stehen für Wasserstoff oder einen C₁-C₆ Alkylrest, der gegebenenfalls ein- oder mehrfach, gleich oder verschieden mit Hydroxy, -C₁-C₃-Alkoxy, -NR⁸R⁹ oder Heterocyclyl substituiert ist, wobei der Heterocylylring ein- oder mehrfach, gleich oder verschieden substituiert sein kann mit C₁-C₃-Alkyl oder -C(O)-R⁷,
oder
- R⁵ und R⁶: bilden alternativ gemeinsam mit dem Stickstoffatom einen 5- bis 7-gliedrigen Ring, der gegebenenfalls zusätzlich zum Stickstoffatom ein weiteres Heteroatom enthält und der gegebenenfalls mit C₁-C₆-Alkyl und/oder mit -C(O)-R⁷ ein- oder mehrfach, gleich oder verschieden substituiert ist.

Bevorzugt stehen R⁵ und R⁶ unabhängig voneinander für Wasserstoff oder einen C₁-C₆ Alkylrest, der gegebenenfalls ein- oder mehrfach, gleich oder verschieden mit Hydroxy, -NR⁸R⁹ oder C₁-C₃-Alkoxy substituiert sein kann.

Besonders bevorzugt stehen R⁵ und R⁶ unabhängig voneinander für Wasserstoff oder einen C₁-C₄-Alkylrest.

In der allgemeinen Formel (I) kann R⁷ stehen für einen C₁-C₆ Alkylrest.

Bevorzugt steht R⁷ für einen C₁-C₄-Alkylrest.

Besonders bevorzugt steht R⁷ für einen C₁-C₃-Alkylrest.

In der allgemeinen Formel (I) können R⁸, R⁹, R¹⁰ unabhängig voneinander stehen für Wasserstoff oder einen C₁-C₆-Alkylrest.

Bevorzugt stehen R⁸ und R⁹ unabhängig voneinander für Wasserstoff oder einen C₁-C₄-Alkylrest und R¹⁰ für Wasserstoff.

Besonders bevorzugt stehen R⁸ und R⁹ unabhängig voneinander für Wasserstoff oder einen C₁-C₃-Alkylrest und R¹⁰ für Wasserstoff.

In der allgemeinen Formel (I) können R¹¹ und R¹² unabhängig voneinander stehen für: Wasserstoff oder einen C₁-C₆ Alkylrest, der gegebenenfalls ein- oder mehrfach, gleich oder verschieden mit Hydroxy, -C₁-C₃-Alkoxy, -NR⁸R⁹ oder Heterocyclyl substituiert ist, wobei der Heterocylylring ein- oder mehrfach, gleich oder verschieden substituiert sein kann mit C₁-C₃-Alkyl oder -C(O)-R⁷.

Bevorzugt stehen R¹¹ und R¹² unabhängig voneinander für Wasserstoff oder einen C₁-C₆ Alkylrest, der gegebenenfalls ein- oder mehrfach, gleich oder verschieden mit Morpholin oder durch -N(CH₃)₂, -NH-CH₃ oder -NH-C₂H₅ substituiert sein kann.

Eine bevorzugte Untergruppe bilden Verbindungen der Formel (I) mit den Bausteinen A, B, C und D, in der die Bausteine B und D in ortho-Stellung zueinander stehen und
- Q₁: für einen Pyridinyl-, Pyridazinyl-, Pyrimidinyl-, Pyrazinyl- oder Triazinylring steht, und
- R¹ und R²: unabhängig voneinander stehen für
(i) Wasserstoff, Hydroxy, Halogen, Cyano, -CF₃, -NR⁵R⁶ oder
(ii) einen C₁-C₆-Alkyl-, C₁-C₆-Alkoxy-, C₁-C₆-Fluoralkyl- oder einen C₁-C₆-Fluoralkoxyrest oder
(iii) -SO₂-R⁷, und
- Q₂: für einen Phenyl-, Thienyl-, Thiazolyl-, Furanyl-, Pyrrolyl-, Oxazolyl-, Imidazolyl-, Pyrazolyl-, Isoxazolyl, Isothiazolyl, 1,2,3-Triazolyl, Pyridinyl-, Pyridazinyl-, Pyrimidinyl-, Pyrazinyl-, 1,2,3,4-Tetrahydro-isochinolinyl-, 1,2,3,4-Tetrahydro-chinolinyl-, 2,3-Dihydro-1H-indolyl-, 2,3-Dihydro-1H-isoindolyl-, 4,5,6,7-Tetrahydro-thieno[2,3-c]pyridinyl-, 4,5,6,7-Tetrahydro-thieno[3,2-c]pyridinyl-, 2,3-Dihydro-benzofuranyl-, Benzo[1,3]dioxolyl-, 2,3-Dihydro-benzo[1,4]dioxinyl-, 1,2,3,4-Tetrahydro-chinoxalinyl- oder einen 3,4-Dihydro-2H-benzo[1,4]oxazinylring steht, und
- R³ und R⁴: unabhängig voneinander stehen für
(i) Wasserstoff, Halogen, -NR¹¹R¹² oder
(ii) einen C₁-C₆-Alkyl- oder C₁-C₆-Alkoxyrest,
jeweils gegebenenfalls mit Morpholin oder -NR⁸R⁹ substituiert, wobei
- R⁵ und R⁶: unabhängig voneinander stehen für Wasserstoff oder einen C₁-C₆ Alkylrest, der gegebenenfalls ein- oder mehrfach, gleich oder verschieden mit Hydroxy, -NR⁸R⁹ oder C₁-C₃-Alkoxy substituiert sein kann, und
- R⁷: für einen C₁-C₄ Alkylrest steht, und
- R⁸ und R⁹: unabhängig voneinander stehen für Wasserstoff oder einen C₁-C₄ Alkylrest, und
- R¹¹ und R¹²: unabhängig voneinander stehen für Wasserstoff oder einen C₁-C₆ Alkylrest, der gegebenenfalls ein- oder mehrfach, gleich oder verschieden mit Morpholin oder durch -N(CH₃)₂, -NH-CH₃ oder -NH-C₂H₅ substituiert sein kann,
sowie deren Salze, Enantiomere und Diastereomere.

Besonders bevorzugt sind Verbindungen der allgemeinen Formel (I) mit den Bausteinen A, B, C und D, in der die Bausteine B und D in ortho-Stellung zueinander stehen und
- Q₁: für einen Pyridyl- oder Pyrazinylring steht, und
- R¹ und R²: unabhängig voneinander stehen für
(i) Wasserstoff, -NH₂, Hydroxy, Halogen, -CF₃, oder
(ii) einen C₁-C₆-Alkoxy-, C₁-C₆-Fluoralkyl- oder einen C₁-C₆-Fluoralkoxyrest, und
- Q₂: für einen Phenyl-, Pyrazolyl-, Thienyl-, Pyridinyl- oder 4,5,6,7-Tetrahydro-thieno[2,3-c]pyridinylring steht, und
- R³ und R⁴: unabhängig voneinander stehen für Wasserstoff, Halogen oder C₁-C₆-Alkyl,
sowie deren Salze, Enantiomere und Diastereomere.

### Erstellung der erfindungsgemäßen Verbindungen

### Verfahrensvariante 1:

### a) Herstellung von Intermediaten der Formel (III)

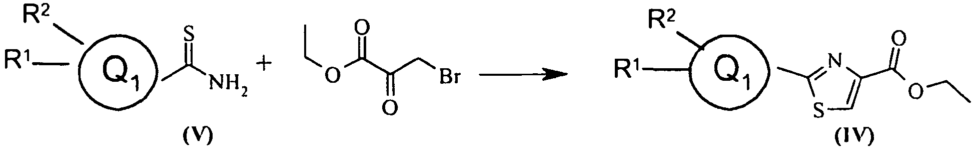

Intermediate der allgemeinen Formel (III) können in zwei Synthesestufen hergestellt werden. Zuerst werden Intermediate der Formel (V) und Brombrenztraubensäureethylester durch Erhitzen in organischen Lösemitteln wie Toluol zu Intermediaten der Formel (IV) überführt.

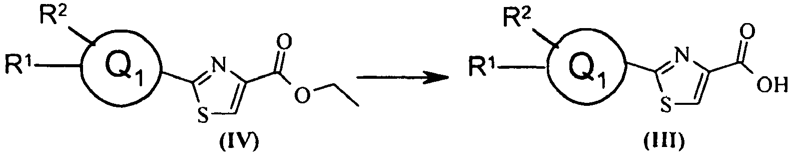

Danach kann der Ethylester (IV) verseift werden, was zu den Intermediaten (III) führt. Hierbei haben Q₁, R¹ und R² die in der allgemeinen Formel (I) gemäß der Ansprüche 1 bis 7 angegebenen Bedeutungen.

### b) Herstellung des Endproduktes

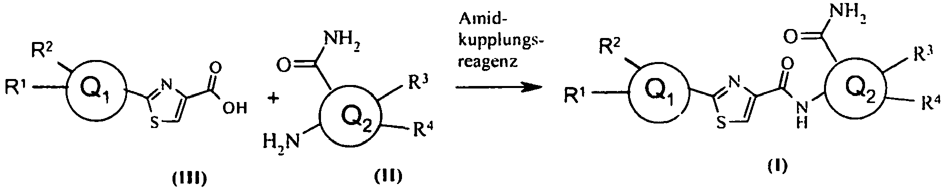

Die Darstellung der erfindungsgemäßen Verbindungen der allgemeinen Formel (I) kann durch Umsetzung der Intermediate gemäß Formel (III) mit Verbindungen der Fomel (II) erfolgen durch ein Amidkupplungsreagenz (siehe Publikation Chemfiles, Peptide Synthesis, 2007, 7, Nr. 2 der Firma Sigma-Aldrich). Hierbei können zum Beispiel Carbodiimide (zum Beispiel N-Cyclohexyl-N'-(2-morpholinoethyl)carbodiimid metho-p-toluolsulfonat CAS2491-17-0) oder Uroniumsalze (zum Beispiel O-(7-Azabenzotriazol-1-yl)-*N,N,N'N'*-tetramethyluroniumhexafluorophosphat (HATU)) verwendet werden in Kombination mit einer Base wie zum Beispiel Pyridin.
Hierbei haben Q₁, Q₂, R¹, R², R³ und R⁴ die in der allgemeinen Formel (I) gemäß der Ansprüche 1 bis 7 angegebenen Bedeutungen.

### Verfahrensvariante 2:

### a) Herstellung von Intermediaten der Formel (VI)

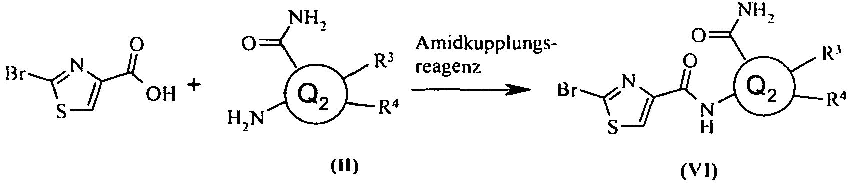

2-Bromthiazol-4-carbonsäure wird mit Intermediaten der Formel (II) durch ein Amidkupplungsreagenzien (siehe Verfahrensvariante 1) zu Intermediaten der Formel (VI) umgesetzt.

### b) Herstellung des Endproduktes

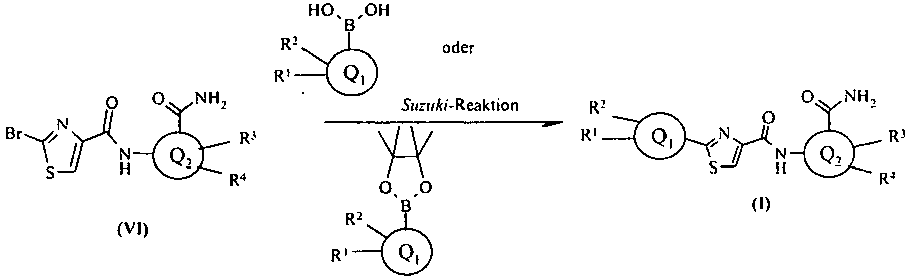

Intermediate der Formel (VI) werden mit Boronsäuren oder Boronsäurepinakolestern im Rahmen einer *Suzuki-Miyaura*-Reaktion zu den erfindungsgemäßen Verbindungen der Formel (I) umgesetzt. Als Katalysatoren bzw. Liganden kommen dabei zum Beispiel die in N. Miyaura, A. Suzuki, Chem. Rev.; 1995; 95(7); 2457-2483 oder in C. J. O'Brien et. al. Chem. Eur. J. 2006, 12, 4743 - 4748 sowie die in der Publikation Chemfiles, Catalysis, 2007, 7, Nr. 5 von der Firma Sigma-Aldrich beschrieben Katalysatoren bzw. Katalysatorensysteme in Betracht.
Hierbei haben Q₁, Q₂, R¹, R², R³ und R⁴ die in der allgemeinen Formel (I) gemäß der Ansprüche 1 bis 7 angegebenen Bedeutungen.

### Reinigung der erfindungsgemäßen Verbindungen

In einigen Fällen können die erfindungsgemäßen Verbindungen durch präparative HPLC zum Beispiel durch ein Autopurifier-Gerät der Firma Waters (Detektion der Verbindungen durch UV-Detektion sowie Elektrospray-lonisation) in Kombination mit kommerziell erhältlichen, vorgepackten HPLC Säulen (zum Beispiel Säule XBridge (Firma Waters), C18, 5µm, 30 x 100mm) gereinigt werden. Als Lösemittelsystem kann Acetonitril / Wasser +0,1% Trifluoressigsäure verwendet werden (Fluss 50 ml / min). Zum Entfernen des HPLC-Lösemittelgemisches kann eine Gefriertrocknung oder eine Zentrifugation erfolgen. Die so erhaltenen Verbindungen können als Trifluoressigsäure (TFA)-Salze vorliegen und können zu den jeweiligen freien Basen durch dem Fachmann bekannte Standard-Laborprozeduren überführt werden.
In einigen Fällen können die erfindungsgemäßen Verbindungen durch Chromatographie an Kieselgel gereinigt werden. Hierbei können zum Beispiel vorgepackte Silica Gel Cartridges (zum Beispiel von der Firma Separtis, *Isolute*® *Flash silica gel*) in Kombination mit dem Flashmaster II Chromatograhiegerät (Argonaut/Biotage) und Chromatographielösemittel bzw. -Gemische wie zum Beispiel Hexan, Essigester sowie Dichlormethan und Methanol in Betracht.

### Strukturanalytik der erfindungsgemäßen Verbindungen:

In einigen Fällen werden die erfindungsgemäßen Verbindungen durch LC-MS analysiert: Retentionszeiten Rₜ aus der LC-MS-Analytik: Detektion: UV = 200 - 400 nm (*Acquity* HPLC-System der Firma *Waters*)/ MS 100-800 Daltons; 20 V (Micromass / *Waters* ZQ 4000) im ESI⁺-Modus (zur Erzeugung positiv geladener Molekülionen); HPLC-Säule: X Bridge (*Waters*), 2.1 x 50 mm, BEH 1.7 µm; Flussmittel: A: H2O/0.05% HC(O)OH, B: CH3CN/0.05% HC(O)OH. Gradient: 10-90% B in 1.7 min, 90% B für 0.2 min, 98-2% B in 0.6 min; Flussrate: 1.3 ml / min.
Die Angabe *LC-MS (ZQ)* bezieht sich auf die Verwendung eines *Waters* ZQ4000 Gerätes und die Angabe *LC-MS (SQD)* auf die Benutzung eines Single Quadrupol API (Atomic Pressure lonization) Massendetektors (*Waters*) zur Aufnahme eines Massenspektrums.

### Verfahrensvariante 1

### Beispiel 1.1

### N-[2-(Aminocarbonyl)phenyl]-2-(3-pyridinyl)-4-thiazolcarboxamid

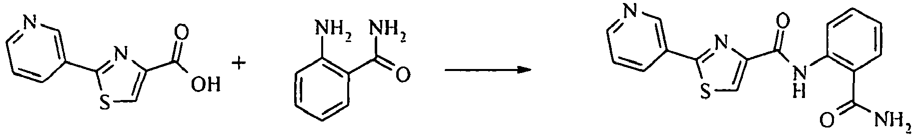

2-(3-Pyridyl)thiazol-4-carbonsäure (206 mg, 1 mmol) und 2-Aminobenzamid (163 mg, 1.2 mmol) werden vorgelegt und man gibt Chloroform (10 ml) und Pyridin (1.2 ml) zu. Dann addiert man 1-Hydroxybenzotriazol Hydrat (230 mg) und 1-Cyclohexyl-3-(2-morpholinoethyl)carbodiimid Metho-P-toluolsulfonat CAS[2491-17-0] (635 mg) und lässt 20 h bei Raumtemperatur rühren. Man saugt ab und wäscht den Feststoff mit Essigester, Wasser und erneut mit Essigester. Nach Reinigung des Feststoffes durch präparative HPLC erhält man Beispiel 1.1 als weißen Schaum (58 mg).

C₁₆H₁₂N₄O₂S, M = 324.4, LC-MS (ZQ): Rₜ = 0.89, m/z = 325 [M+H]⁺. 1H-NMR (400 MHz, D6-DMSO): δ = 7.16 (m, 1H), 7.57 (m, 2H), 7.84 (m, 2H), 8.27 (s, 1H), 8.40 (m, 1H), 8.54 (s, 1H), 8.71 (m, 2H), 9.32 (m, 1H), 13.3 (s, 1H).

### Beispiel 1.2

### N-[5-(Aminocarbonyl)-1-methyl-1H-pyrazol-4-yl]-2-(4-pyridinyl)-4-thiazolcarboxamid

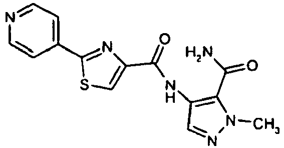

Analog zur Synthese des Beispieles 1.1 erhält man N-[5-(Aminocarbonyl)-1-methyl-1H-pyrazol-4-yl]-2-(4-pyridinyl)-4-thiazolcarboxamid als weißen Schaum durch die Reaktion von 2-(4-Pyridyl)thiazol-4-carbonsäure und 4-Amino-2-methyl-2H-pyrazol-3-carbonsäureamid und nachfolgendes Reinigen per HPLC.

C₁₄H₁₂N₆O₂S, M = 328.4, LC-MS (ZQ): Rₜ = 0.65, m/z = 329 [M+H]⁺.

### Beispiel 1.3

### N-[2-(Aminocarbonyl)phenyl]-2-(4-pyridinyl)- 4-thiazolcarboxamid

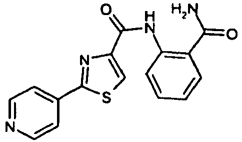

Analog zur Synthese des Beispieles 1.1 erhält man N-[2-(Aminocarbonyl)phenyl]-2-(4-pyridinyl)-4-thiazolcarboxamid als weißen Schaum durch die Reaktion von 2-(4-Pyridyl)thiazol-4-carbonsäure mit 2-Aminobenzamid und nachfolgendes Reinigen per HPLC.

C₁₆H₁₂N₄O₂S, M = 324.4, LC-MS (ZQ): Rₜ = 0.81, m/z = 325 [M+H]⁺. 1H-NMR (300 MHz, D6-DMSO): δ = 7.21 (m, 1H), 7.59 (m, 1H), 7.89 (m, 2H), 8.18 (m, 2H), 8.34 (s, 1H), 8.72 (s, 1H), 8.75 (m, 1H), 8.87 (m, 2H), 13.3 (s, 1H).

### Beispiel 1.4

### N-[2-(Aminocarbonyl)-4-methylphenyl]-2-(3-pyridinyl)-4-thiazolcarboxamid

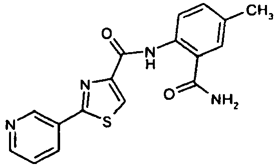

Analog zur Synthese des Beispieles 1.1 erhält man N-[2-(Aminocarbonyl)-4-methylphenyl]-2-(3-pyridinyl)-4-thiazolcarboxamid durch die Reaktion von 2-(3-Pyridyl)thiazol-4-carbonsäure mit 2-Amino-5-methylbenzamid und nachfolgendes Reinigen per HPLC.

C₁₇H₁₄N₄2_{S}, M = 338.4, LC-MS (SQD): Rₜ = 0.87, m/z = 339 [M+H]⁺.

### Beispiel 1.5

### N-[2-(Aminocarbonyl)-5-methylphenyl]-2-(3-pyridinyl)-4-thiazolcarboxamid

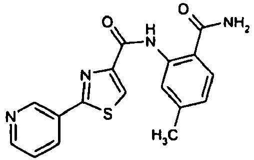

Analog zur Synthese des Beispieles 1.1 erhält man N-[2-(Aminocarbonyl)-5-methylphenyl]-2-(3-pyridinyl)-4-thiazolcarboxamid durch die Reaktion von 2-(3-Pyridyl)thiazol-4-carbonsäure mit 2-Amino-4-methylbenzamid, nachfolgendes Absaugen und Waschen des entstandenen Feststoffes mit Wasser und Essigester sowie Trocknen im Vakuum.

C₁₇H₁₄N₄O₂S, M = 338.4, LC-MS (SQD): Rₜ = 0.87, m/z = 339 [M+H]⁺. 1H-NMR (300 MHz, D6-DMSO): δ = 2.39 (s, 3H), 7.02 (m, 1H), 7.61 (m, 1H), 7.76 - 7.85 (m, 2H), 8.24 (s, 1H), 8.45 (m, 1H), 8.57 (s, 1H), 8.64 (m, 1H), 8.74 (m, 1H), 9.36 (m, 1H), 13.4 (s, 1H).

### Beispiel 1.6

### N-[2-(Aminocarbonyl)-4-chlorophenyl]-2-(3-pyridinyl)-4-thiazolcarboxamid

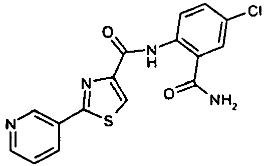

Analog zur Synthese des Beispieles 1.1 erhält man N-[2-(Aminocarbonyl)-4-chlorophenyl]-2-(3-pyridinyl)-4-thiazolcarboxamid als Feststoff durch die Reaktion von 2-(3-Pyridyl)thiazol-4-carbonsäure mit 2-Amino-5-chlorbenzamid und nachfolgendes Reinigen per HPLC.

C₁₆H₁₁CIN₄O₂, 358.8, LC-MS (ZQ): Rₜ = 1.03, m/z = 359 [M+H]⁺.

### Beispiel 1.7

### 3-[(2-Pyridin-3-yl-thiazol-4-carbonyl)amino]pyridin-2-carbonsäureamid

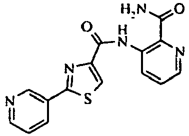

Analog zur Synthese von Beispiel 1.1 erhält man 3-[(2-Pyridin-3-yl-thiazol-4-carbonyl)amino]pyridin-2-carbonsäureamid als Feststoff durch die Reaktion von 2-(3-Pyridyl)thiazol-4-carbonsäure und 3-Amino-pyridin-2-carbonsäureamid und nachfolgendes Reinigen per HPLC.

C₁₅H₁₁N₅O₂S, M = 325.4. LC-MS (ZQ): Rₜ =0.94, m/z = 326 [M+H]⁺.

### Beispiel 1.8

### N-[2-(Aminocarbonyl)-5-methylphenyl]-2-(4-pyridinyl)-4-thiazolcarboxamid

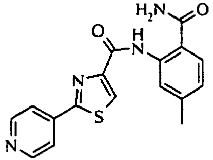

Analog zur Synthese von Beispiel 1.1 erhält man N-[2-(Aminocarbonyl)-5-methylphenyl]-2-(4-pyridinyl)-4-thiazolcarboxamid als Feststoff durch die Reaktion von 2-(4-Pyridyl)thiazol-4-carbonsäure und 2-Amino-4-methylbenzamid und nachfolgendes Reinigen per HPLC.

C₁₇H₁₄N₄O₂S, M = 338.4. LC-MS (ZQ): Rₜ = 0.98, m/z = 339 [M+H]⁺. 1H-NMR (300 MHz, D6-DMSO): δ = 2.39 (s, 3H), 7.03 (m, 1H), 7.75 -7.85 (2H), 8.13 (m, 2H), 8.26 (br. s., 1H), 8.63 (s, 1H), 8.84 (m, 2H), 13.4 (s, 1H).

### Beispiel 1.9

### 6-Methyl-2-[(2-pyridin-4-yl-thiazol-4-carbonyl)-amino]-4,5,6,7-tetrahydro-thieno[2,3-c]pyridin-3-carbonsäureamid

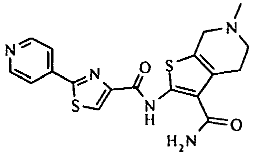

Analog zur Synthese von Beispiel 1.1 erhält man 6-Methyl-2-[(2-pyridin-4-yl-thiazol-4-carbonyl)-amino]-4,5,6,7-tetrahydro-thieno[2,3-c]pyridin-3-carbonsäureamid als Feststoff durch die Reaktion von 2-(4-Pyridyl)thiazol-4-carbonsäure und 2-Amino-6-methyl-4,5,6,7-tetrahydro-thieno[2,3-c]pyridin-3-carbonsäureamid und nachfolgendes Reinigen per HPLC.

C₁₈H₁₇N₅O₂S₂, M = 399.5. LC-MS (ZQ): Rₜ = 0.67, m/z = 400 [M+H]⁺.

### Beispiel 1.10

### 2-Pyridin-4-yl-thiazol-4-carbonsäure(3-carbamoyl-1-methyl-1H-pyrazol-4-yl)-amid

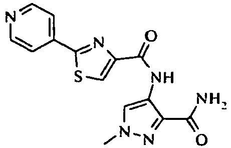

Analog zur Synthese von Beispiel 1.1 erhält man 2-Pyridin-4-yl-thiazol-4-carbonsäure(3-carbamoyl-1-methyl-1H-pyrazol-4-yl)-amid als Feststoff durch die Reaktion von 2-(4-Pyridyl)thiazol-4-carbonsäure und 4-Amino-1-methyl-1H-pyrazol-3-carbonsäureamid und nachfolgendes Reinigen per HPLC.

C₁₄H₁₂N₆O₂S, M = 328.1. LC-MS (ZQ): Rₜ = 0.72, m/z = 329 [M+H]⁺. 1H-NMR (400 MHz, D6-DMSO): δ = 3.90 (s, 3H), 7.55 (br. s., 1H), 7.76 (br. s, 1H), 8.01 (d, 2H), 8.34 (s, 1H), 8.63 (s, 1H), 8.80 (d, 2H), 11.5 (s, 1H).

### Beispiel 1.11

### N-[2-(Aminocarbonyl)-5-methylphenyl]-2-[4-(trifluoromethyl)-3-pyridinyl]- 4-thiazolcarboxamid

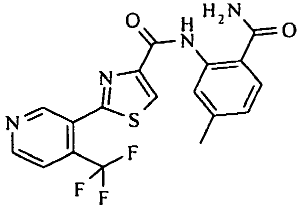

### a) Herstellung des Intermediates III.1

### 2-(4-Trifluoromethyl-pyridin-3-yl)-thiazol-4-carbonsäure

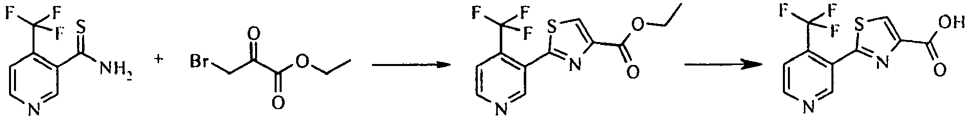

4-(Trifluormethyl)pyridin-3-thiocarboxamid (201 mg),
Brombrenztraubensäureethylester (122 Microliter) und Toluol (5 ml) werden zusammengegeben und 4 Stunden am Rückfluss erhitzt. Das Toluol wird entfernt und der Rückstand mit Dichlormethan aufgenommen.
Die organische Phase wird mit Wasser und gesättigter Kochsalzlösung gewaschen und über Natriumsulfat getrocknet.
Nach Entfernen des Lösemittels am Rotationsverdampfer erhält man einen Feststoff (266 mg), der mit Tetrahydrofuran (THF) (2 ml), Ethanol (2 ml) und 1M Natronlauge (4 ml) versetzt wird. Danach wird 3.5 h bei Raumtemperatur gerührt und mit 1 M Salzsäurelösung auf pH 2 eingestellt.
Die organischen Lösemittel werden weitestgehend entfernt und der Rückstand mit Essigester versetzt. Die organische Phase wird mit Wasser und gesättigter Kochsalzlösung gewaschen, über Natriumsulfat getrocknet und eingeengt. Man erhält Intermediat III.1 als braunen Feststoff (221 mg).

C₁₀H₅F₃N₂O₂S, M = 264.0, LC-MS (ZQ): Rₜ = 0.83, m/z = 275 [M+H]⁺. 1H-NMR (300 MHz, D6-DMSO): δ = 7.99 (d, 1H), 8.74 (s, 1H), 9.01 - 9.07 (2H), 13.3 (br. s, 1H).

### b) Herstellung des Endproduktes

Analog zur Synthese des Beispieles 1.1 erhält man N-[2-(Aminocarbonyl)-5-methylphenyl]-2-[4-(trifluoromethyl)-3-pyridinyl]- 4-thiazolcarboxamid als Feststoff durch die Reaktion des Intermediates III.1 und 2-Amino-4-methylbenzamid und nachfolgendes Reinigen per HPLC.

C₁₈H₁₃F₃N₄O₂S, M = 406.4, LC-MS (ZQ): Rₜ = 1.14, m/z = 407 [M+H]⁺.

### Beispiel 1.12

### N-[2-(Aminocarbonyl)-5-methylphenyl]-2-[6-(2,2,2-trifluoroethoxy)-3-pyridinyl]-4-thiazolcarboxamid

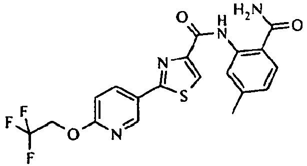

### a) Herstellung des Intermediates III.2

### 2-(6-Trifluorethoxypyridin-3-yl)-thiazol-4-carbonsäure

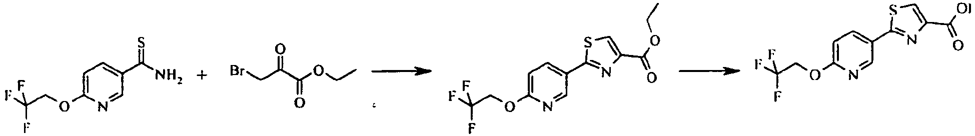

Ausgehend von 6-(2,2,2-Trifluorethoxy)pyridin-3-thiocarboxamid CAS175277-59-5 und Brombrenztraubensäureethylester erhält man 2-(6-Trifluorethoxypyridin-3-yl)-thiazol-4-carbonsäure als Feststoff analog zur Synthese von Intermediat III.1.

C₁₁H₇F₃N₂O₃S, M = 304.0, LC-MS (ZQ): Rₜ = 1.09, m/z = 305 [M+H]⁺. 1H-NMR (400 MHz, D6-DMSO): δ = 5.02 (q, 2H, C*H*₂CF₃), 7.12 (d, 1H), 8.31 (dd, 1H), 8.48 (s, 1H), 8.77 (d, 1H).

### b) Herstellung des Endproduktes

Analog zur Synthese des Beispieles 1.1 erhält man N-[2-(Aminocarbonyl)-5-methylphenyl]-2-[6-(2,2,2-trifluoroethoxy)-3-pyridinyl]-4-thiazolcarboxamid als Feststoff durch die Reaktion des Intermediates III.2 und 2-Amino-4-methylbenzamid und nachfolgendes Reinigen per HPLC.

C₁₉H₁₅F₃N₄O₃S, M = 436.4, LC-MS (ZQ): Rₜ = 1.03, m/z = 437 [M+H]⁺.

### Beispiel 1.13

### N-[2-(Aminocarbonyl)-5-methylphenyl]-2-(6-methoxy-3-pyridinyl)-4-thiazolcarboxamid

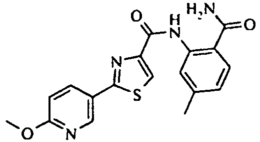

### a) Herstellung des Intermediates III.3

### 2-(6-Methoxypyridin-3-yl)-thiazol-4-carbonsäure

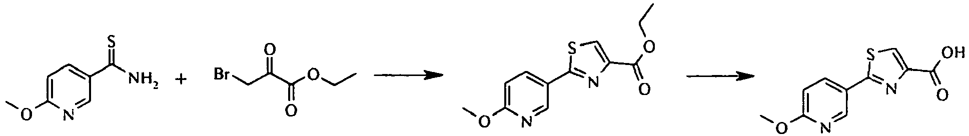

Ausgehend von 6-Methoxypyridin-3-carbothioamid CAS175277-49-3 und Brombrenztraubensäureethylester erhält man 2-(6-Methoxypyridin-3-yl)-thiazol-4-carbonsäure als Rohprodukt analog zur Synthese des Intermediates III.1.

C₁₀H₈N₂O₃S, M = 236.3, LC-MS: Rₜ = 2.40, m/z = 237 [M+H]⁺. Aufnahme durch ein Autopurifier HPLC-System der Firma *Waters* (HPLC 2525 Binary Gradient Modul (Waters), Detektor Micromass ZQ, *Waters,* UV Lampe Photo Diode Array 2996, Waters, Wellenlänge 210 - 350 nm, Säule XBridge 4.6x50mm C18 3.5µm, Gradient von Acetonitril 1% bis 99% (0.1 % Trifluoressigsäure (TFA), Runtime = 8 min Fluß = 2.00 ml/min)

### b) Herstellung des Endproduktes

Analog zur Synthese der Beispiel 1.1 erhält man N-[2-(Aminocarbonyl)-5-methylphenyl]-2-(6-methoxy-3-pyridinyl)-4-thiazolcarboxamid als Feststoff durch die Reaktion des Intermediates III.3 und 2-Amino-4-methylbenzamid und nachfolgendes Reinigen per HPLC.

C₁₈H₁₆N₄O₃S, M = 368.4. LC-MS (ZQ): Rₜ = 1.20, m/z = 369 [M+H]⁺.

### Beispiel 1.14

### N-[3-(Aminocarbonyl)-1-methyl-1H-pyrazol-4-yl]-2-(pyrazin-2-yl)-4-thiazolcarboxamid

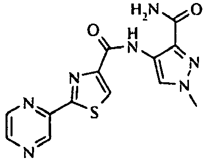

### a) Herstellung des Intermediates III.4

### 2-(Pyrazin-2-yl)-thiazol-4-carbonsäure

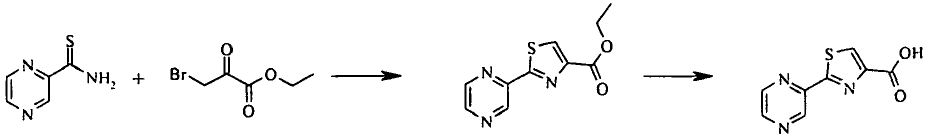

Ausgehend von Pyrazin-2-carbothioamid und Brombrenztraubensäureethylester erhält man analog zur Synthese von Intermediat III.1 2-(Pyrazin-2-yl)-thiazol-4-carbonsäure (Intermediat III.4) als Rohprodukt.

C₈H₅N₃O₂S, M = 207.2, LC-MS (ZQ): Rₜ = 0.66, m/z = 208 [M+H]⁺. 1H-NMR (400 MHz, D6-DMSO, ausgewählte Signale): δ = 8.58 (m, 1H), 8.63 (d, 1H), 9.41 (m, 1H).

### b) Herstellung des Endproduktes

Analog zur Synthese von Beispiel 1.1 erhält man N-[3-(Aminocarbonyl)-1-methyl-1H-pyrazol-4-yl]-2-(pyrazin-2-yl)-4-thiazolcarboxamid als Feststoff durch die Reaktion von Intermediat III.4 und 4-Amino-1-methyl-1H-pyrazol-3-carbonsäureamid und nachfolgendes Reinigen per HPLC.

C₁₃H₁₁N₇O₂S, M = 329.1. LC-MS (ZQ): Rₜ = 0.94, m/z = 330 [M+H]⁺. 1H-NMR (400 MHz, D6-DMSO): δ = 3.91 (s, 3H), 7.57 (br. s., 1H), 7.77 (br. s, 1H), 8.33 (s, 1H), 8.62 (s, 1H), 8.75 (m, 1H), 8.80 (m, 1H), 9.35 (m, 1H), 11.5 (s, 1H).

### Beispiel 1.15

### 6-Methyl-2-[(2-pyrazin-2-yl-thiazol-4-carbonyl)-amino]-4,5,6,7-tetrahydro-thieno[2,3-c]pyridin-3-carbonsäureamid

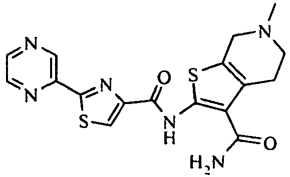

Analog zur Synthese von Beispiel 1.1 erhält man
6-Methyl-2-[(2-pyrazin-2-yl-thiazol-4-carbonyl)-amino]-4,5,6,7-tetrahydro-thieno[2,3-c]pyridin-3-carbonsäureamid als Feststoff durch die Reaktion von Intermediat III.4 und 2-Amino-6-methyl-4,5,6,7-tetrahydro-thieno[2,3-c]pyridin-3-carbonsäureamid und nachfolgendes Reinigen per HPLC.

C₁₇H₁₆N₆O₂S₂, M = 400.1. LC-MS (ZQ): Rₜ = 0.76, m/z = 401 [M+H]⁺.

### Beispiel 1.16

### N-[5-(Aminocarbonyl)-1-methyl-1H-pyrazol-4-yl]-2-(3-pyridinyl)-4-thiazolcarboxamid

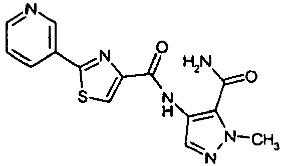

Herstellung von N-[5-(Aminocarbonyl)-1-methyl-1H-pyrazol-4-yl]-2-(3-pyridinyl)-4-thiazolcarboxamid mittels Amidkupplung durch HATU:
2-(3-Pyridyl)thiazol-4-carbonsäure (82 mg, 0.4 mmol) und 4-Amino-2-methyl-2H-pyrazol-3-carbonsäureamid (56 mg, 0.4 mmol) werden in Chloroform (3 ml) und Pyridin (0.2 ml) vorgelegt. Dann addiert man HATU O-(7-Azabenzotriazol-1-yl)-N,N,N',N'-tetramethyluronium hexafluoro-phosphat (228 mg) und lässt bei Raumtemperatur 70 h im geschlossenen Gefäß rühren. Der ausgefallene Feststoff wird abgesaugt, mit Essigester, Wasser und erneut mit Essigester gewaschen und im Vakuum getrocknet. Nach Reinigung des Feststoffes durch HPLC wird Beispiel 1.16 als weißer Schaum erhalten.

C₁₄H₁₂N₆O₂S, M = 328.4, LC-MS (ZQ): Rₜ = 0.72, m/z = 329 [M+H]⁺. 1H-NMR (300 MHz, D6-DMSO): δ = 3.99 (s, 3H), 7.58 (m, 1H), 7.85 (br. s., 2H), 7.93 (s, 1H), 8.36 (m, 1H), 8.53 (m, 1H), 8.70 (m, 1H), 9.24 (m, 1H), 10.7 (s, 1H).

### Beispiel 1.17

### N-[2-(Aminocarbonyl)-5-(1,1-dimethylethyl)-3-thienyl]-2-(3-pyridinyl)-4-thiazolcarboxamid

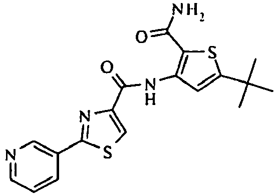

Analog zur Synthese des Beispieles 1.16 erhält man N-[2-(Aminocarbonyl)-5-(1,1-dimethylethyl)-3-thienyl]-2-(3-pyridinyl)-4-thiazolcarboxamid als Feststoff durch die Reaktion von 2-(3-Pyridyl)thiazol-4-carbonsäure mit 3-Amino-5-(tert-butyl)thiophen-2-carboxamid und Reinigung per HPLC.

C₁₈H₁₈N₄O₂S₂, M = 386.5. 1H-NMR (400 MHz, D6-DMSO): δ = 1.35 (s, 9H), 7.59 (m, 1H), 7.63 (breites s, 2H), 7.99 (s, 1H), 8.39 (m, 1H), 8.55 (m, 1H), 8.71 (m, 1H), 9.28 (m, 1H), 12.9 (s, 1H).

### Beispiel 1.18

### N-[2-(Aminocarbonyl)-4-fluorophenyl]-2-(3-pyridinyl)-4-thiazolcarboxamid

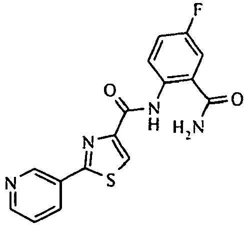

Analog zur Synthese des Beispieles 1.16 erhält man N-[2-(Aminocarbonyl)-4-fluorophenyl]-2-(3-pyridinyl)-4-thiazolcarboxamid als Feststoff durch die Reaktion von 2-(3-Pyridyl)thiazol-4-carbonsäure mit 2-Amino-5-fluorbenzamid und Reinigung per HPLC.

C₁₆H₁₁FN₄O₂S, M = 342.3. 1H-NMR (400 MHz, D6-DMSO): δ = 7.44 (m, 1H), 7.58 (m, 1H), 7.71 (m, 1H), 8.02 (s, 1H), 8.34 (s, 1H), 8.41 (m, 1H), 8.56 (s, 1H), 8.70 (m, 1H), 8.76 (m, 1H), 9.32 (m, 1H), 13.2 (s, 1H).

### Beispiel 1.19

### N-[2-(Aminocarbonyl)-3-thienyl]-2-(3-pyridinyl)-4-thiazolcarboxamid

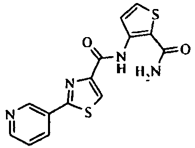

Analog zur Synthese des Beispieles 1.16 erhält man N-[2-(Aminocarbonyl)-3-thienyl]-2-(3-pyridinyl)-4-thiazolcarboxamid als Feststoff durch die Reaktion von 2-(3-Pyridyl)thiazol-4-carbonsäure mit 3-Aminothiophen-2-carboxamid und Reinigung per HPLC.

C₁₈H₁₈N₄O₂S₂, M = 330.0. LC-MS (ZQ): Rₜ = 0.90, m/z = 331 [M+H]⁺. 1H-NMR (300 MHz, D6-DMSO): δ = 7.65 (m, 1H), 7.78 (br. s., 2H), 7.81 (d, 1H), 8.17 (d, 1H), 8.46 (m, 1H), 8.63 (s, 1H), 8.76 (m, 1H), 9.34 (m, 1H), 13.0 (s, 1H).

### Beispiel 1.20

### N-[3-(Aminocarbonyl)-4-methyl-2-thienyl]-2-(3-pyridinyl)-4-thiazolcarboxamid

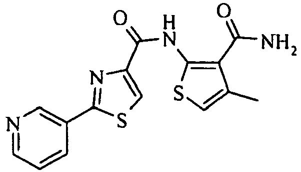

Analog zur Synthese des Beispieles 1.16 erhält man N-[3-(Aminocarbonyl)-4-methyl-2-thienyl]-2-(3-pyridinyl)-4-thiazolcarboxamid als Feststoff durch die Umsetzung von 2-(3-Pyridyl)thiazol-4-carbonsäure mit 2-Amino-4-methylthiophen-3-carboxamid und Reinigung per HPLC.

C₁₅H₁₂N₄O₂S₂, M = 344.1. LC-MS (ZQ): Rₜ = 0.97, m/z = 345 [M+H]⁺. 1H-NMR (300 MHz, D6-DMSO): δ = 2.37 (s, 3H), 6.72 (s, 1H), 7.61 (m, 1H), 8.39 (m, 1H), 8.62 (s, 1H), 8.72 (m, 1H), 9.26 (m, 1H), 13.2 (s, 1H). 13C-NMR (100 MHz, D6-DMSO): δ = 17.22, 114.5, 117.9, 124.9, 127.7, 128.6, 132.9, 134.6, 145.5, 147.3, 149.2, 151.8, 157.3, 165.3, 168.2.

### Beispiel 1.21

### 3-[(2-Pyridin-4-yl-thiazol-4-carbonyl)-amino]-pyridin-2-carbonsäureamid

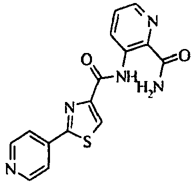

Analog zur Synthese des Beispieles 1.16 erhält man 3-[(2-Pyridin-4-yl-thiazol-4-carbonyl)-amino]-pyridin-2-carbonsäureamid als Feststoff durch die Umsetzung von 2-(4-Pyridyl)thiazol-4-carbonsäure mit 3-Aminopyridin-2-carbonsäureamid (Berrie et. al., J. Chem. Soc. 1952, 2042) und Reinigung per HPLC.

1H-NMR (400 MHz, D6-DMSO): δ = 7.65 (m, 1H), 8.02 (m, 2H), 8.12 (br. s., 1H), 8.35 (m, 1H), 8.52 (br. s., 1H), 8.68 (s, 1H), 8.77 (m, 2H), 9.18 (m, 1H), 13.8 (s, 1H). C₁₅H₁₁N₅O₂S, M = 325.4. LC-MS (ZQ): Rₜ = 0.85, m/z = 326 [M+H]⁺.

### Beispiel 1.22

### 3-[(2-Pyridin-4-yl-thiazol-4-carbonyl)-amino]-isonicotinamid

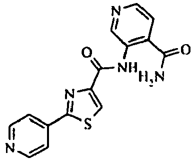

Analog zur Synthese des Beispieles 1.16 erhält man 3-[(2-Pyridin-4-yl-thiazol-4-carbonyl)-amino]-isonicotinamid als Feststoff durch die Umsetzung von 2-(4-Pyridyl)thiazol-4-carbonsäure mit 3-Aminoisonicotinamid und Reinigung per HPLC.

C₁₅H₁₁N₅O₂S, M = 325.4. LC-MS (ZQ): Rₜ = 0.74, m/z = 326 [M+H]⁺.

### Verfahrensvariante 2

### Beispiel 2.1

### N-[2-(Aminocarbonyl)phenylJ-2-(6-methoxy-3-pyridinyl)-4-thiazolcarboxamid

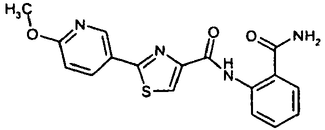

### a) Herstellung des Intermediates VI.1

### N-[2-(Aminocarbonyl)phenyl]-2-brom-4-thiazolcarboxamid

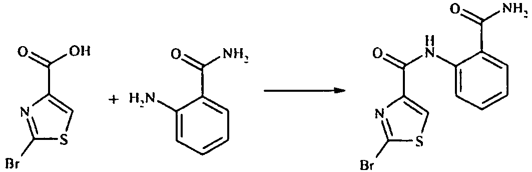

2-Brom-4-thiazolcarbonsäure (1 g), HATU (2.01 g) und 2-Aminobenzamid (0.65 g) werden in N,N-Dimethylformamid (DMF) (20 ml) vorgelegt. Man kühlt mit einem Eisbad ab und gibt N,N-DÜsopropylethylamin (0.90 ml) zu. Man lässt 6 Tage bei Raumtemperatur rühren, gießt das Reaktionsgemisch auf Eiswasser, lässt unter Rühren auftauen und saugt den ausgefallenen Feststoff ab, wäscht zweimal mit Wasser, zweimal mit Diethylether und trocknet im Vakuum. Man erhält das Intermediat VI.1 als Feststoff (1.4 g).

C₁₁H₈BrN₃O₂S, M = 326.2. 1H-NMR (300 MHz, D6-DMSO): δ = 7.20 (m, 1H), 7.56 (m, 1H), 7.79 (s, 1H), 7.84 (m, 1H), 8.30 (s, 1H), 8.47 (m, 1H), 8.67 (m, 1H), 12.9 (s, 1H).

### b) Herstellung des Endproduktes

Das Intermediat VI.1 (65 mg, 0.2 mmol) und 2-Methoxypyridin-5-boronsäure (43 mg, 0.28 mmol) werden in Toluol (1.5 ml), Ethanol (1.5 ml) und Natriumcarbonatlösung (180 Microliter, 2M) in einem Microwellengefäß vorgelegt. Danach addiert man Pd(PPh₃)₄ (23 mg, 0.1 Äquivaltente) und erhitzt 20 min bei 120°C (300 Watt) in der Microwelle (Gerät CEM Explorer der Firma CEM). Man verdünnt mit Wasser, extrahiert mit Essigester, engt die organische Phase in der Zentrifuge ein und reinigt durch HPLC.

C₁₇H₁₄N₄O₃S, M = 354.4, Rₜ = 1.09, m/z = 355 [M+H]⁺.

### Beispiel 2.2

### N-[2-(Aminocarbonyl)phenyl]-2-(2-methoxy-3-pyridinyl)-4-thiazolcarboxamid

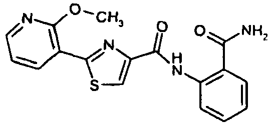

Analog Beispiel 2.1 erhält man N-[2-(Aminocarbonyl)phenyl]-2-(2-methoxy-3-pyridinyl)-4-thiazolcarboxamid als Feststoff aus Intermediat VI.1 und 2-Methoxypyridin-3-boronsäure.

C₁₇H₁₄N₄O₃S, M = 354.4, LC-MS (ZQ): Rₜ = 1.12, m/z = 355 [M+H]⁺.

### Beispiel 2.3

### N-[2-(Aminocarbonyl)phenyl]-2-(5-chlor-3-pyridinyl)-4-thiazolcarboxamid

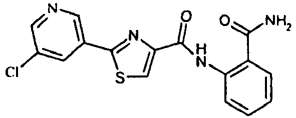

Analog Beispiel 2.5 erhält man N-[2-(Aminocarbonyl)phenyl]-2-(5-chlor-3-pyridyl)-4-thiazolcarboxamid aus Intermediat VI.1 und 5-Chlor-3-pyridylboronsäure.

C₁₆H₁₁CIN₄O₂S, M = 358.8. LC-MS (ZQ): Rₜ = 1.08, m/z = 359 [M+H]⁺.

### Beispiel 2.4

### N-[2-(Aminocarbonyl)-5-methylphenyl]-2-(6-amino-pyridin-3-yl)-thiazol-4-carbonsäureamid

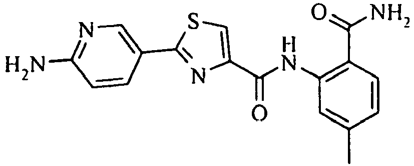

### a) Herstellung des Intermediates VL2

### N-[2-(Aminocarbonyl)-5-methylphenyl]-2-brom-4-thiazolcarboxamid

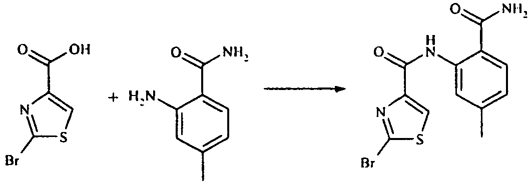

Analog zur Synthese von Intermediat VI.1 wird 2-Brom-4-thiazolcarbonsäure und 2-Amino-4-methylbenzamid zu N-[2-(Aminocarbonyl)-5-methylphenyl]-2-brom-4-thiazolcarboxamid umgesetzt.

C₁₂H₁₀BrN₃O₂S, M = 339.0, LC-MS (ZQ): Rₜ = 1.04, m/z = 340 [M+H]⁺. 1H-NMR (300 MHz, D6-DMSO): δ = 2.32 (s, 3H), 6.97 (m, 1H) 7.65 (br. s., 1H), 7.70 (d, 1H), 8.18 (br. s., 1H), 8.40(s, 1H), 8.49 (m, 1H), 13.0 (s, 1H).

### b) Herstellung des Endproduktes

Das Intermediat VI.2 (150 mg) und 2-Amino-5-(4,4,5,5-tetramethyl-1,3,2-dioxaborolan-2-yl)pyridin (CAS827614-64-2) (136 mg) werden in Dimethylethylenglycol (3 ml) und wässriger Natriumhydrogencarbonatlösung (2 ml, 1 M) vorgelegt und danach wird Pd(PPh₃)₄ (102 mg, 0.2 Äquivalente) zugegeben und die Mischung bei 110°C 45 min in der Microwelle (CEM Explorer, 300 Watt) erhitzt. Man verdünnt mit Wasser und extrahiert mit Essigester, engt die organische Phase ein und reinigt den Rückstand durch HPLC.

C₁₇H₁₅N₅O₂S, M = 353.1, LC-MS (ZQ): Rₜ = 0.79, m/z = 354 [M+H]⁺.
1H-NMR (400 MHz, D6-DMSO, ausgewählte Signale): δ = 2.33 (s, 3H), 6.79 (d, 1H), 6.96 (m, 1H), 7.67 (s, 1H), 7.73 (d, 1H), 8.56 (m, 1H), 8.62 (m, 1H), 13.2 (s, 1H).

### Beispiel 2.5

### N-[2-(Aminocarbonyl)-5-methylphenyl]-2-(6-hydroxy-pyridin-3-yl)-thiazol-4-carbonsäureamid

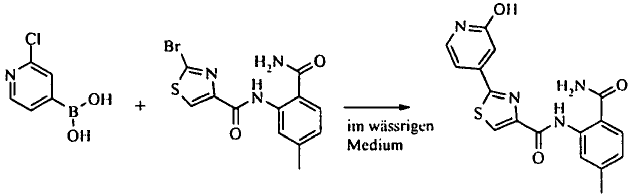

Das Intermediat VI.2 (150 mg) und 2-Chlorpyridin-4-boronsäure (84 mg) werden in Dimethylglycol (3 ml) und wässriger Natriumhydrogencarbonatlösung (2 ml, 1 M) vorgelegt. Man gibt Pd(PPh₃)₄ (51 mg) zu und erwärmt 30 min bei 110°C in der Mikrowelle (300W) (Gerät CEM Explorer). Danach wird erneut Pd(PPh₃)₄ (52 mg) zugegeben und 30 min bei 110°C in der Microwelle erhitzt. Nach Aufarbeitung wie bei Beispiel 2.1 und Reinigung per HPLC erhält man N-[2-(Aminocarbonyl)-5-methylphenyl]-2-(6-hydroxy-pyridin-3-yl)-thiazol-4-carbonsäureamid (9.8 mg) als weißen Feststoff.

C₁₇H₁₄N₄O₃S, M = 354.1, LC-MS (ZQ): Rₜ = 1.25, m/z = 355 [M+H]⁺.

### TLR induzierte Zytokinfreisetzung in humanen "peripheral blood mononuclear cells" (PBMC)

### Versuchsprinzip

Aus humanem Vollblut isolierte PBMCs werden mit einem TLR Liganden stimuliert. Die Zytokin-Bestimmung wird mittels ELISA-Kits, eine Proliferations-/Zellstoffwechselbestimmung wird mit Alamarblue durchgeführt.

### PBMC-Isolierung:

Für die Zellpräparation werden ca. 200 ml Blut mit einem Antikoagulanz (z.B. Citrat-Monovetten) versetzt. Pro Leucosep-Röhrchen werden 15ml Histopaque (Raumtemperatur, RT) eingefüllt und durch kurzes Anzentrifugieren (eine Minute bei 1000 x g, RT) durch die eingesetzte Fritte nach unten gedrückt. In die so vorbereiteten Röhrchen werden 20ml Blut gegeben und für 15 Minuten bei 800xg (RT) zentrifugiert. Nach der Zentrifugation ergibt sich von oben nach unten folgende Schichtung:
Plasma - PBMC - Histopaque - Filterscheibe - Histopaque - Erythrozyten und Granulozyten. Das überstehende Plasma wird abgesaugt. Die PBMC werden mitsamt dem darunterliegenden Histopaque in ein neues 50ml Röhrchen überführt, wobei immer der Inhalt von zwei Leucosep-Röhrchen
in ein 50ml Röhrchen gegeben wird. Die 50ml Röhrchen werden dann mit PBS auf 50ml aufgefüllt. Diese Zellsuspension wird für zehn Minuten bei 300xg (RT) zentrifugiert.
Der flüssige Überstand wird abgekippt und das Zellpellet mit wenig PBS resuspendiert und anschließend mit PBS auf 50ml aufgefüllt. Dieser Waschschritt wird zweimal wiederholt. Das entstandene Pellet wird in einem definierten Volumen von Medium (mit Zusätzen) aufgenommen. Zur Testung der Substanzen werden PBMC mit titrierten Konzentrationen der Testsubstanzen z.B. in Gegenwart oder Abwesenheit von TLR7 oder TLR9 Liganden für 18 Stunden inkubiert. Am nächsten Tag werden die Überstände mittels spezifischer ELISA auf den Gehalt von TNF-alpha oder anderen Zyto- oder Chemokinen untersucht. Die Stoffwechselaktivität der behandelten Zellen wird mithilfe von Alamarblue bestimmt.

### Ergebnisse:

| **Beispiel** | **EC₅₀ (TNF-α TLR7)** |
|---|---|
| 1.1 | 9.0e-7 mol/L |
| 1.2 | 2.4e-6 mol/L |
| 1.3 | 1.3e-6 mol/L |
| 1.4 | 4.9e-7 mol/L |
| 1.5 | 4.1 e-6 mol/L |
| 1.6 | 3.0-e-5 mol/L |
| 1.7 | 1.8e-6 mol/L |
| 1.8 | 1.1 e-6 mol/L |
| 1.11 | 3.0e-5 mol/L |
| 1.17 | 2.6e-6 mol/L |
| 1.18 | 1.9e-6 mol/L |
| 1.21 | 5.2e-6 mol/L |
| 1.22 | 7.0e-6 mol/L |
| 2.4 | 1.3e-6 mol/L |

## Patentansprüche

1. Verbindungen der allgemeinen Formel (I) mit den Bausteinen A, B, C und D , 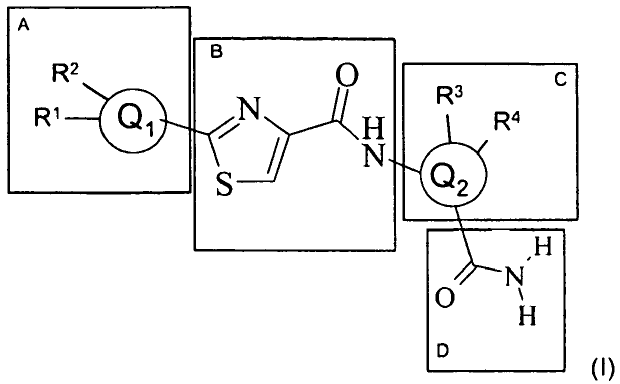 in der
die Bausteine B und D in ortho-Stellung zueinander stehen und
Q₁ für einen Heteroarylring mit 6 Ringatomen steht, und
R¹ und R² unabhängig voneinander stehen für
(i) Wasserstoff, Hydroxy, Nitro, Halogen, Cyano, -CF₃, -NR⁵R⁶ oder
(ii) -C(O)-OR¹⁰, -C(O)-R⁷, -C(O)-C₁-C₃-Fluoralkyl, -C(O)-NR⁵R⁶, -NH-C(O)-R⁷ oder
(iii) einen C₁-C₆-Alkyl-, C₂-C₆ -Alkenyl-, C₂-C₆-Alkinyl-, C₁-C₆-Alkoxy-, C₁-C₆-Fluoralkyl- oder einen C₁-C₆-Fluoralkoxyrest,
jeweils gegebenenfalls mit -C₁-C₃-Alkoxy, Hydroxy, -C(O)-OR¹⁰ oder -NR⁸R⁹ ein- oder mehrfach, gleich oder verschieden substituiert, oder
(iv) -O-SO₂-NR⁵R⁶, -SO₂-R⁷, -SO₂-NR⁵R⁶, und
Q₂ für einen Aryl-, Heteroaryl- oder einen hydrierten bizyklischen Heteroarylring steht, und
R³ und R⁴ unabhängig voneinander stehen für
(i) Wasserstoff, Halogen oder-NR¹¹R¹², oder
(ii) einen C₁-C₆-Alkyl- oder C₁-C₆-Alkoxyrest, die jeweils gegebenenfalls ein- oder mehrfach, gleich oder verschieden mit Hydroxy, C₁-C₃-Alkoxy, -NR⁸R⁹ oder Heterocyclyl substituiert sind, wobei der Heterocylylring ein- oder mehrfach, gleich oder verschieden substituiert sein kann mit C₁-C₃-Alkyl oder -C(O)-R⁷,
wobei R⁵ und R⁶ unabhängig voneinander stehen für Wasserstoff oder einen C₁-C₆ Alkylrest, der gegebenenfalls ein- oder mehrfach, gleich oder verschieden mit Hydroxy, -C₁-C₃-Alkoxy, -NR⁸R⁹ oder Heterocyclyl substituiert ist, wobei der Heterocylylring ein- oder mehrfach, gleich oder verschieden substituiert sein kann mit C₁-C₃-Alkyl oder -C(O)-R⁷, oder
R⁵ und R⁶ bilden alternativ gemeinsam mit dem Stickstoffatom einen 5- bis 7-gliedrigen Ring, der gegebenenfalls zusätzlich zum Stickstoffatom ein weiteres Heteroatom enthält und der gegebenenfalls mit C₁-C₆-Alkyl und/oder mit -C(O)-R⁷ ein- oder mehrfach, gleich oder verschieden substituiert ist, und
R⁷ für einen C₁-C₆ Alkylrest steht, und
R⁸, R⁹, R¹⁰ unabhängig voneinander stehen für Wasserstoff oder einen C₁-C₆ Alkylrest, und
R¹¹ und R¹² unabhängig voneinander stehen für Wasserstoff oder einen C₁-C₆ Alkylrest, der gegebenenfalls ein- oder mehrfach, gleich oder verschieden mit Hydroxy, -C₁-C₃-Alkoxy, -NR⁸R⁹ oder Heterocyclyl substituiert ist, wobei der Heterocylylring ein- oder mehrfach, gleich oder verschieden substituiert sein kann mit C₁-C₃-Alkyl oder -C(O)-R⁷,
sowie deren Salze, Enantiomere und Diastereomere.

2. Verbindungen der allgemeinen Formel (I) mit den Bausteinen A, B, C und D, in der
die Bausteine B und D in ortho-Stellung zueinander stehen und
Q₁ für einen Pyridinyl-, Pyridazinyl-, Pyrimidinyl-, Pyrazinyl- oder Triazinylring steht, und
R¹ und R² unabhängig voneinander stehen für
(i) Wasserstoff, Hydroxy, Halogen, Cyano, -CF₃, -NR⁵R⁶ oder
(ii) einen C₁-C₆-Alkyl-, C₁-C₆-Alkoxy-, C₁-C₆-Fluoralkyl- oder einen C₁-C₆-Fluoralkoxyrest oder
(iii) -SO₂-R⁷, und
Q₂ für einen Phenyl-, Thienyl-, Thiazolyl-, Furanyl-, Pyrrolyl-, Oxazolyl-, Imidazolyl-, Pyrazolyl-, Isoxazolyl, Isothiazolyl, 1,2,3-Triazolyl, Pyridinyl-, Pyridazinyl-, Pyrimidinyl-, Pyrazinyl-, 1,2,3,4-Tetrahydro-isochinolinyl-, 1,2,3,4-Tetrahydro-chinolinyl-, 2,3-Dihydro-1H-indolyl-, 2,3-Dihydro-1H-isoindolyl-, 4,5,6,7-Tetrahydro-thieno[2,3-c]pyridinyl-, 4,5,6,7-Tetrahydro-thieno[3,2-c]pyridinyl-, 2,3-Dihydro-benzofuranyl-, Benzo[1,3]dioxolyl-, 2,3-Dihydro-benzo[1,4]dioxinyl-, 1,2,3,4-Tetrahydro-chinoxalinyl- oder einen 3,4-Dihydro-2H-benzo[1,4]oxazinylring steht, und
R³ und R⁴ unabhängig voneinander stehen für
(i) Wasserstoff, Halogen, -NR¹¹R¹² oder
(ii) einen C₁-C₆-Alkyl- oder C₁-C₆-Alkoxyrest, jeweils gegebenenfalls mit Morpholin oder -NR⁸R⁹ substituiert,
wobei
R⁵ und R⁶ unabhängig voneinander stehen für Wasserstoff oder einen C₁-C₆ Alkylrest, der gegebenenfalls ein- oder mehrfach, gleich oder verschieden mit Hydroxy, -NR⁸R⁹, oder C₁-C₃-Alkoxy substituiert sein kann, und
R⁷ für einen C₁-C₄ Alkylrest steht, und
R⁸ und R⁹ unabhängig voneinander stehen für Wasserstoff oder einen C₁-C₄ Alkylrest,
R¹¹ und R¹² unabhängig voneinander stehen für Wasserstoff oder einen C₁-C₆ Alkylrest, der gegebenenfalls ein- oder mehrfach, gleich oder verschieden mit Morpholin oder durch -N(CH₃)₂. -NH-CH₃ oder -NH-C₂H₅ substituiert sein kann,
sowie deren Salze, Enantiomere und Diastereomere.

3. Verbindungen der allgemeinen Formel (I) mit den Bausteinen A, B, C und D gemäß Anspruch 1 oder 2, in der
Q₁ für einen Pyridyl- oder Pyrazinylring steht,
sowie deren Salze, Enantiomere und Diastereomere.

4. Verbindungen der allgemeinen Formel (I) mit den Bausteinen A, B, C und D gemäß einem der Ansprüche 1 bis 3, in der
R¹ und R² unabhängig voneinander stehen für
(i) Wasserstoff, -NH₂, Hydroxy, Halogen, -CF₃, oder
(ii) einen C₁-C₆-Alkoxy-, C₁-C₆-Fluoralkyl- oder einen C₁-C₆-Fluoralkoxyrest,
sowie deren Salze, Enantiomere und Diastereomere.

5. Verbindungen der allgemeinen Formel (I) mit den Bausteinen A, B, C und D gemäß einem der Ansprüche 1 bis 4, in der
Q₂ für einen Phenyl-, Pyrazolyl-, Thienyl-, Pyridinyl- oder 4,5,6,7-Tetrahydro-thieno[2,3-c]pyridinylring steht,
sowie deren Salze, Enantiomere und Diastereomere.

6. Verbindungen der allgemeinen Formel (I) mit den Bausteinen A, B, C und D gemäß einem der Ansprüche 1 bis 5, in der
R³ und R⁴ unabhängig voneinander stehen für Wasserstoff, Halogen oder C₁-C₆-Alkyl,
sowie deren Salze, Enantiomere und Diastereomere.

7. Verbindungen der allgemeinen Formel (I) mit den Bausteinen A, B, C und D in der
die Bausteine B und D in ortho-Stellung zueinander stehen und
Q₁ für einen Pyridyl- oder Pyrazinylring steht, und
R¹ und R² unabhängig voneinander stehen für
(i) Wasserstoff, -NH₂, Hydroxy, Halogen, -CF₃, oder
(ii) einen C₁-C₆-Alkoxy-, C₁-C₆-Fluoralkyl- oder einen C₁-C₆-Fluoralkoxyrest
Q₂ für einen Phenyl-, Pyrazolyl-, Thienyl-, Pyridinyl- oder 4,5,6,7-Tetrahydro-thieno[2,3-c]pyridinylring steht, und
R³ und R⁴ unabhängig voneinander stehen für Wasserstoff, Halogen oder C₁-C₆-Alkyl,
sowie deren Salze, Enantiomere und Diastereomere.

8. Verbindungen nach einem der Ansprüche 1 bis 7, nämlich
N-[2-(Aminocarbonyl)phenyl]-2-(3-pyridinyl)-4-thiazolcarboxamid
N-[5-(Aminocarbonyl)-1-methyl-1H-pyrazol-4-yl]-2-(4-pyridinyl)-4-thiazolcarboxamid
N-[2-(Aminocarbonyl)phenyl]-2-(4-pyridinyl)- 4-thiazolcarboxamid
N-[2-(Aminocarbonyl)-4-methylphenyl]-2-(3-pyridinyl)-4-thiazolcarboxamid
N-[2-(Aminocarbonyl)-5-methylphenyl]-2-(3-pyridinyl)-4-thiazolcarboxamid
N-[2-(Aminocarbonyl)-4-chlorophenyl]-2-(3-pyridinyl)-4-thiazolcarboxamid
3-[(2-Pyridin-3-yl-thiazol-4-carbonyl)amino]pyridin-2-carbonsäureamid
N-[2-(Aminocarbonyl)-5-methylphenyl]-2-(4-pyridinyl)-4-thiazolcarboxamid
6-Methyl-2-[(2-pyridin-4-y)-thiazol-4-carbonyl)-amino]-4,5,6,7-tetrahydrothieno[2,3-c]pyridin-3-carbonsäureamid
2-Pyridin-4-yl-thiazol-4-carbonsäure(3-carbamoyl-1-methyl-1H-pyrazol-4-yl)-amid
N-[2-(Aminocarbonyl)-5-methylphenyl]-2-[4-(trifluoromethyl)-3-pyridinyl]- 4-thiazolcarboxamid
N-[2-(Aminocarbonyl)-5-methylphenyl]-2-[6-(2,2,2-trifluoroethoxy)-3-pyridinyl]-4-thiazolcarboxamid
N-[2-(Aminocarbonyl)-5-methylphenyl]-2-(6-methoxy-3-pyridinyl)-4-thiazolcarboxamid
N-[3-(Aminocarbonyl)-1-methyl-1H-pyrazol-4-y!]-2-(pyrazin-2-yl)-4-thiazolcarboxamid
6-Methyl-2-[(2-pyrazin-2-yl-thiazol-4-carbonyl)-amino]-4,5,6,7-tetrahydrothieno[2,3-c]pyridin-3-carbonsäureamid
N-[5-(Aminocarbonyl)-1-methyl-1H-pyrazol-4-yl]-2-(3-pyridinyl)-4-thiazolcarboxamid
N-[2-(Aminocarbonyl)-5-(1,1-dimethylethyl)-3-thienyl]-2-(3-pyridinyl)-4-thiazolcarboxamid
N-[2-(Aminocarbonyl)-4-fluorophenyl]-2-(3-pyridinyl)-4-thiazolcarboxamid
N-[2-(Aminocarbonyl)-3-thienyl]-2-(3-pyridinyl)-4-thiazolcarboxamid
N-[3-(Aminocarbonyl)-4-methyl-2-thienyl]-2-(3-pyridinyl)-4-thiazolcarboxamid
3-[(2-Pyridin-4-yl-thiazol-4-carbonyl)-amino]-pyridin-2-carbonsäureamid
3-[(2-Pyridin-4-yl-thiazol-4-carbonyl)-amino]-isonicotinamid
N-[2-(Aminocarbonyl)phenyl]-2-(6-methoxy-3-pyridinyl)-4-thiazolcarboxamid
N-[2-(Aminocarbonyl)phenyl]-2-(2-methoxy-3-pyridinyl)-4-thiazolcarboxamid
N-[2-(Aminocarbonyl)phenyl]-2-(5-chlor-3-pyridinyl)-4-thiazolcarboxamid
N-[2-(Aminocarbonyl)-5-methylphenyl]-2-(6-amino-pyridin-3-yl)-thiazol-4-carbonsäureamid
N-[2-(Aminocarbonyl)-5-methylphenyl]-2-(6-hydroxy-pyridin-3-yl)-thiazol-4-carbonsäureamid

9. Verfahren zur Herstellung von Verbindungen gemäß einem der Ansprüche 1 bis 8 **gekennzeichnet durch** die Schritte
a) Überführung von Intermediaten der Formel (V) zu Intermediaten der Formel (IV) **durch** Umsetzung mit Brombrenztraubensäureethylester durch Erhitzen in organischen Lösemitteln, 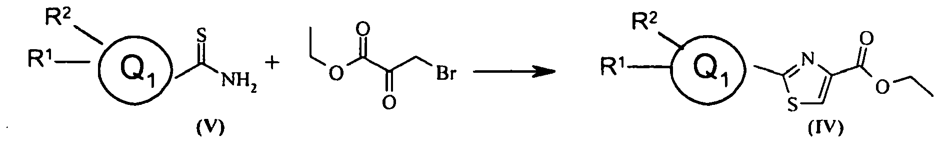
b) Verseifung des Ethylesters (IV) unter Erhalt von Intermediaten der Formel (III), 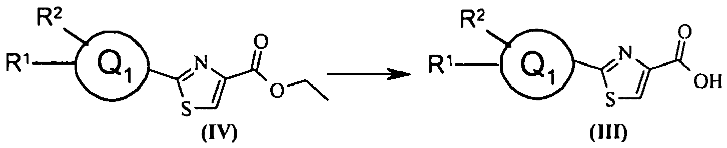
c) Umsetzung der Intermediate gemäß Formel (III) mit Verbindungen der Fomel (II) **durch** ein Amidkupplungsreagenz 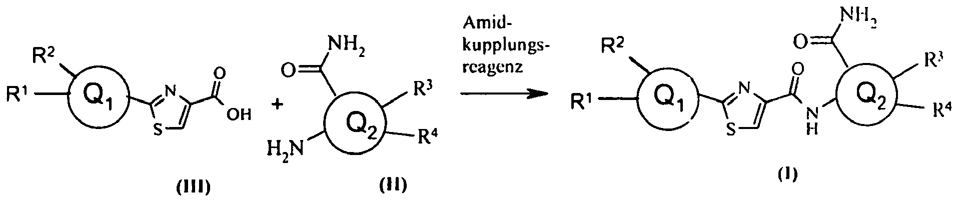 wobei
Q₁, Q₂, R¹, R², R³ und R⁴ die in der allgemeinen Formel (I) gemäß der Ansprüche 1 bis 7 angegebenen Bedeutungen besitzen.

10. Verfahren zur Herstellung von Verbindungen gemäß einem der Ansprüche 1 bis 8 **gekennzeichnet durch** die Schritte
a) Umsetzung von 2-Bromthiazol-4-carbonsäure mit Intermediaten der Formel (II) **durch** ein Amidkupplungsreagenz 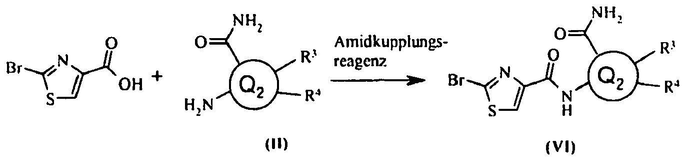
b) Umsetzung der Intermediate der Formel (VI) mit Boronsäuren oder Boronsäurepinakolestern im Rahmen einer *Suzuki*-*Miyaura*-*Reaktion* zu Verbindungen der Formel (I) 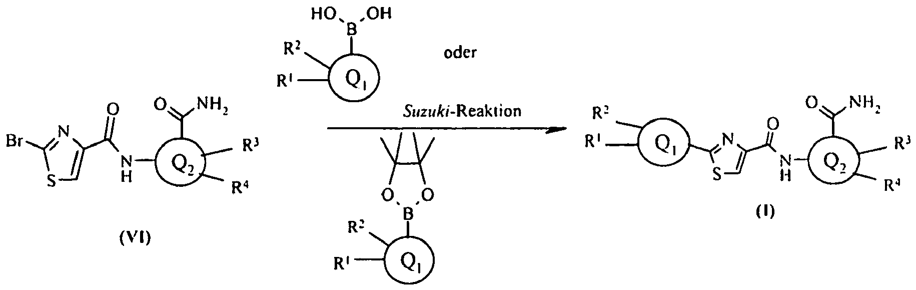 wobei
Q₁, Q₂, R¹, R², R³ und R⁴ die in der allgemeinen Formel (I) gemäß der Ansprüche 1 bis 7 angegebenen Bedeutungen besitzen.

11. Verbindungen gemäß einem der Ansprüche 1 bis 8 zur Verwendung als Arzneimittel.

12. Verbindungen gemäß einem der Ansprüche 1 bis 8 zur Verwendung als Arzneimittel gegen Erkrankungen, die mit entzündlichen, allergischen und / oder proliferativen Prozessen einhergehen.

13. Pharmazeutische Formulierung enthaltend eine Verbindung gemäß einem der Ansprüche 1 bis 8.

## Claims

1. Compounds of the general formula (I) with building blocks A, B, C and D 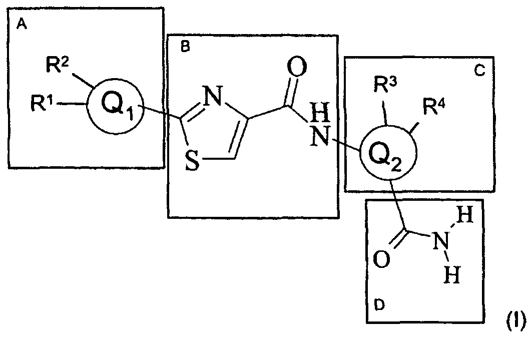 in which
the building blocks B and D are in ortho position relative to one another, and
Q₁ is a heteroaryl ring having 6 ring atoms, and
R¹ and R² are independently of one another
(i) hydrogen, hydroxyl, nitro, halogen, cyano, -CF₃, -NR⁵R⁶ or
(ii) -C(O)-R¹⁰, -C(O)-R⁷, -C(O)-C₁-C₃-fluoroalkyl, -C(O)-NR⁵R⁶, -NH-C(O)-R⁷ or
(iii) a C₁-C₆-alkyl, C₂-C₆-alkenyl, C₂-C₆-alkynyl, C₁-C₆-alkoxy, C₁-C₆-fluoroalkyl or a C₁-C₆-fluoroalkoxy radical,
in each case optionally substituted one or more times, identically or differently, by C₁-C₃-alkoxy, hydroxyl, -C(O)-R¹⁰ or -NR⁸R⁹, or
(iv) -O-SO₂--NR⁵R⁶, -SO₂-R⁷, -SO₂-NR⁵R⁶, and
Q₂ is an aryl, heteroaryl or a hydrogenated bicyclic heteroaryl ring; and
R³ and R⁴ are independently of one another
(i) hydrogen, halogen or -NR¹¹R¹², or
(ii) a C₁-C₆-alkyl or C₁-C₆-alkoxy radical which are in each case optionally substituted one or more times, identically or differently, by hydroxyl, C₁-C₃-alkoxy, -NR⁸R⁹ or heterocyclyl, where the heterocyclyl ring may be substituted one or more times, identically or differently, by C₁-C₃-alkyl or -C(O)-R⁷,
where R⁵ and R⁶ are independently of one another hydrogen or a C₁-C₆-alkyl radical which is optionally substituted one or more times, identically or differently, by hydroxy, -C₁-C₃-alkoxy, -NR⁸R⁹ or heterocyclyl, where the heterocyclyl ring may be substituted one or more times, identically or differently, by C₁-C₃-alkyl or -C(O)-R⁷, or
R⁵ and R⁶ form alternatively together with the nitrogen atom a 5- to 7-membered ring which optionally comprises a further heteroatom in addition to the nitrogen atom, and which is optionally substituted one or more times, identically or differently, by C₁-C₆-alkyl and/or by -C(O)-R⁷, and
R⁷ is a C₁-C₆-alkyl radical, and
R⁸, R⁹, R¹⁰ are independently of one another hydrogen or a C₁-C₆-alkyl radical, and
R¹¹ and R¹² are independently of one another hydrogen or a C₁-C₆-alkyl radical which is optionally substituted one or more times, identically or differently, by hydroxyl, -C₁-C₃-alkoxy, -NR⁸R⁹ or heterocyclyl, where the heterocyclyl ring may be substituted one or more times, identically or differently, by C₁-C₃-alkyl or -C(O)-R⁷,
and the salts, enantiomers and diastereomers thereof.

2. Compounds of the general formula (I) with building blocks A, B, C and D,
in which
the building blocks B and D are in ortho position relative to one another, and
Q₁ is a pyridinyl, pyridazinyl, pyrimidinyl, pyrazinyl or triazinyl ring, and
R¹ and R² are independently of one another
(i) hydrogen, hydroxyl, halogen, cyano, -CF₃, -NR⁵R⁶ or
(ii) a C₁-C₆-alkyl, C₁-C₆-alkoxy, C₁-C₆-fluoroalkyl or a C₁-C₆-fluoroalkoxy radical or
(iii) -SO₂R⁷, and
Q₂ is phenyl, thienyl, thiazolyl, furanyl, pyrrolyl, oxazolyl, imidazolyl, pyrazolyl, isoxazolyl, isothiazolyl, 1,2,3-triazolyl, pyridinyl, pyridazinyl, pyrimidinyl, pyrazinyl, 1,2,3,4-tetrahydroisoquinolinyl, 1,2,3,4-tetrahydroquinolinyl, 2,3-dihydro-1H-indolyl, 2,3-dihydro-1H-isoindolyl, 4,5,6,7-tetrahydrothieno-[2,3-c]pyridinyl, 4,5,6,7-tetrahydrothieno[3,2-c]pyridinyl, 2,3-dihydrobenzofuranyl, benzo[1,3]dioxolyl, 2,3-dihydrobenzo[1,4]dioxinyl, 1,2,3,4-tetrahydroquinoxalinyl or a 3,4-dihydro-2H-benzo[1,4]oxazinyl ring, and
R³ and R⁴ are independently of one another
(i) hydrogen, halogen, -NR¹¹R¹² or
(ii) a C₁-C₆-alkyl or C₁-C₆-alkoxy radical,
in each case optionally substituted by morpholine or -NR⁸R⁹,
where R⁵ and R⁶ are independently of one another hydrogen or a C₁-C₆ alkyl radical which may optionally be substituted one or more times, identically or differently, by hydroxy, -NR⁸R⁹ or C₁-C₃-alkoxy, and
R⁷ is a C₁-C₄ alkyl radical, and
R⁸ and R⁹ are independently of one another hydrogen or a C₁-C₄ alkyl radical,
R¹¹ and R¹² are independently of one another hydrogen or a C₁-C₆ alkyl radical which may optionally be substituted one or more times, identically or differently, by morpholine or by -N(CH₃)₂, -NH-CH₃ or -NH-C₂H₅,
and the salts, enantiomers and diastereomers thereof.

3. Compounds of the general formula (I) with building blocks A, B, C and D according to Claim 1 or 2, in which
Q₁ is a pyridyl or pyrazinyl ring,
and the salts, enantiomers and diastereomers thereof.

4. Compounds of the general formula (I) with building blocks A, B, C and D according to any of Claims 1 to 3, in which
R¹ and R² are independently of one another
(i) hydrogen, -NH₂, hydroxy, halogen, -CF₃, or
(ii) a C₁-C₆-alkoxy, C₁-C₆-fluoroalkyl or a C₁-C₆-fluoroalkoxy radical,
and the salts, enantiomers and diastereomers thereof.

5. Compounds of the general formula (I) with building blocks A, B, C and D according to any of Claims 1 to 4, in which
Q₂ is a phenyl, pyrazolyl, thienyl, pyridinyl or 4,5,6,7-tetrahydro-thieno[2,3-c]pyridinyl ring,
and the salts, enantiomers and diastereomers thereof.

6. Compounds of the general formula (I) with building blocks A, B, C and D according to any of Claims 1 to 5, in which
R³ and R⁴ are independently of one another hydrogen, halogen or C₁-C₆-alkyl,
and the salts, enantiomers and diastereomers thereof.

7. Compounds of the general formula (I) with building blocks A, B, C and D in which
the building blocks B and D are in ortho position relative to one another, and
Q₁ is a pyridyl or pyrazinyl ring, and
R¹ and R² are independently of one another
(i) hydrogen, -NH₂, hydroxy, halogen, -CF₃, or
(ii) a C₁-C₆-alkoxy, C₁-C₆-fluoroalkyl or a C₁-C₆-fluoroalkoxy radical
Q₂ is a phenyl, pyrazolyl, thienyl, pyridinyl or 4,5,6,7-tetrahydrothieno[2,3-c]pyridinyl ring, and
R³ and R⁴ are independently of one another hydrogen, halogen or C₁-C₆-alkyl,
and the salts, enantiomers and diastereomers thereof.

8. Compounds according to any of Claims 1 to 7, namely
N-[2-(aminocarbonyl)phenyl]-2-(3-pyridinyl)-4-thiazolecarboxamide
N-[5-(aminocarbonyl)-1-methyl-1H-pyrazol-4-yl]-2-(4-pyridinyl)-4-thiazolecarboxamide
N-[2-(aminocarbonyl)phenyl]-2-(4-pyridinyl)-4-thiazolecarboxamide
N-[2-(aminocarbonyl)-4-methylphenyl]-2-(3-pyridinyl)-4-thiazolecarboxamide
N-[2-(aminocarbonyl)-5-methylphenyl]-2-(3-pyridinyl)-4-thiazolecarboxamide
N-[2-(aminocarbonyl)-4-chlorophenyl]-2-(3-pyridinyl)-4-thiazolecarboxamide
3-[(2-pyridin-3-ylthiazole-4-carbonyl)amino]pyridine-2-carboxamide
N-[2-(aminocarbonyl)-5-methylphenyl]-2-(4-pyridinyl)-4-thiazolecarboxamide
6-methyl-2-[(2-pyridin-4-ylthiazole-4-carbonyl)amino]-4,5,6,7-tetrahydrothieno[2,3-c]pyridine-3-carboxamide
2-pyridin-4-ylthiazole-4-(3-carbamoyl-1-methyl-1H-pyrazol-4-yl)-carboxamide
N-[2-(aminocarbonyl)-5-methylphenyl]-2-[4-(trifluoromethyl)-3-pyridinyl]-4-thiazolecarboxamide
N-[2-(aminocarbonyl)-5-methylphenyl]-2-[6-(2,2,2-trifluoroethoxy)-3-pyridinyl]-4-thiazolecarboxamide
N-[2-(aminocarbonyl)-5-methylphenyl]-2-(6-methoxy-3-pyridinyl)-4-thiazolecarboxamide
N-[3-(aminocarbonyl)-1-methyl-1H-pyrazol-4-yl]-2-(pyrazin-2-yl)-4-thiazolecarboxamide
6-methyl-2-[(2-pyrazin-2-ylthiazole-4-carbonyl)amino]-4,5,6,7-tetrahydrothieno[2,3-c]pyridine-3-carboxamide
N-[5-(aminocarbonyl)-1-methyl-1H-pyrazol-4-yl]-2-(3-pyridinyl)-4-thiazolecarboxamide
N-[2-(aminocarbonyl)-5-(1,1-dimethylethyl)-3-thienyl]-2-(3-pyridinyl)-4-thiazolecarboxamide
N-[2-(aminocarbonyl)-4-fluorophenyl]-2-(3-pyridinyl)-4-thiazolecarboxamide
N-[2-(aminocarbonyl)-3-thienyl]-2-(3-pyridinyl)-4-thiazolecarboxamide
N-[3-(aminocarbonyl)-4-methyl-2-thienyl]-2-(3-pyridinyl)-4-thiazolecarboxamide
3-[(2-pyridin-4-ylthiazole-4-carbonyl)amino]pyridine-2-carboxamide
3-[(2-pyridin-4-ylthiazole-4-carbonyl)amino]isonicotinamide
N-[2-(aminocarbonyl)phenyl]-2-(6-methoxy-3-pyridinyl)-4-thiazolecarboxamide
N-[2-(aminocarbonyl)phenyl]-2-(2-methoxy-3-pyridinyl)-4-thiazolecarboxamide
N-[2-(aminocarbonyl)phenyl]-2-(5-chloro-3-pyridinyl)-4-thiazolecarboxamide
N-[2-(aminocarbonyl)-5-methylphenyl]-2-(6-aminopyridin-3-yl)-thiazole-4-carboxamide
N-[2-(aminocarbonyl)-5-methylphenyl]-2-(6-hydroxypyridin-3-yl)-thiazole-4-carboxamide.

9. Process for preparing compounds according to any of Claims 1 to 8 **characterized by** the steps
a) conversion of intermediates of the formula (V) into intermediates of the formula (IV) by reaction with ethyl bromopyruvate by heating in organic solvents 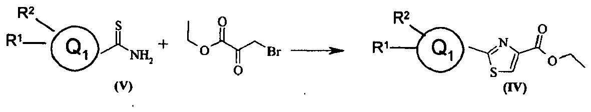
b) hydrolysis of the ethyl ester (IV) to obtain intermediates of the formula (III) 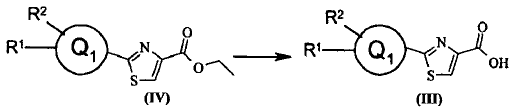
c) reaction of the intermediates of formula (III) with compounds of the formula (II) by an amide coupling reagent 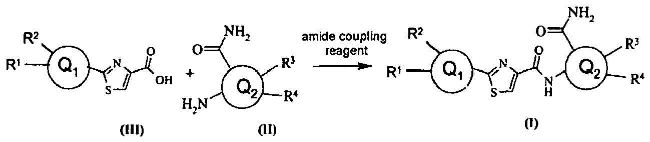 where
Q₁, Q₂, R¹, R², R³ and R⁴ have the meanings indicated in the general formula (I) according to Claims 1 to 7.

10. Process for preparing compounds according to any of Claims 1 to 8 **characterized by** the steps
a) reaction of 2-bromothiazole-4-carboxylic acid with intermediates of the formula (II) by an amide coupling reagent 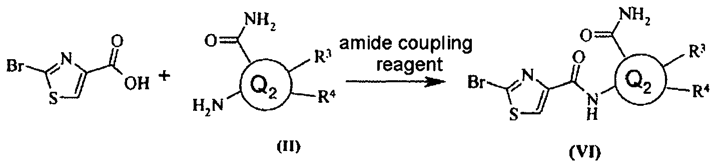
b) reaction of the intermediates of the formula (VI) with boronic acids or boronic acid pinacol esters in a *Suzuki*-*Miyaura* reaction to give compounds of the formula (I) 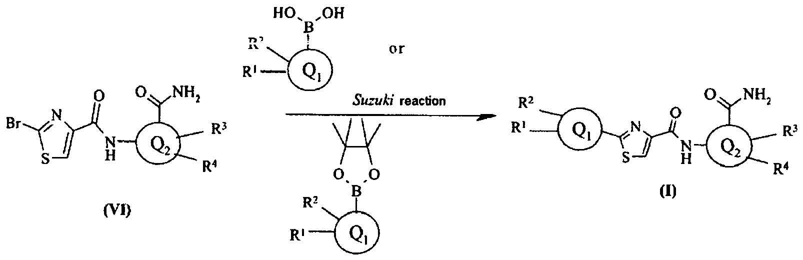 where
Q₁, Q₂, R¹, R², R³ and R⁴ have the meanings indicated in the general formula (I) according to Claims 1 to 7.

11. Compounds according to any of Claims 1 to 8 for use as pharmaceuticals.

12. Compounds according to any of Claims 1 to 8 for use as pharmaceuticals for disorders associated with inflammatory, allergic and/or proliferative processes.

13. Pharmaceutical formulation comprising a compound according to any of Claims 1 to 8.

## Revendications

1. Composés de formule générale (I) présentant les éléments constitutifs A, B, C et D, 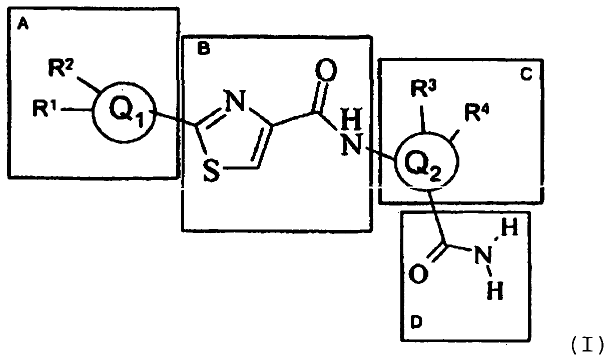 dans laquelle
les éléments constitutifs B et D se trouvent en position ortho l'un par rapport à l'autre et
Q₁ représente un cycle hétéroaryle à 6 atomes formant le cycle, et
R¹ et R² représentent indépendamment l'un de l'autre
(i) un atome d'hydrogène ou d'halogène, ou un groupe hydroxy, nitro, cyano, -CF₃, -NR⁵R⁶ ou
(ii) -C(O)-OR¹⁰, -C(O)-R⁷, -C(O)-fluoroalkyle(C₁-C₃), -C(O)-NR⁵R⁶, -NH-C(O)-R⁷ ou
(iii) un radical alkyle en C₁-C₆, alcényle en C₂-C₆, alcynyle en C₂-C₆, alcoxy en C₁-C₆, fluoroalkyle(C₁-C₆), ou un radical fluoroalcoxy(C₁-C₆),
éventuellement portant chacun un ou plusieurs substituants, identiques ou différents, alcoxy en C₁-C₃, hydroxy, -C(O)-OR¹⁰ ou -NR⁸R⁹, ou
(iv) -O-SO₂-NR⁵R⁶, -SO₂-R⁷, -SO₂NR⁵R⁶ et
Q₂ représente un cycle aryle, hétéroaryle ou un cycle hétéroaryle bicyclique hydrogéné, et
R³ et R⁴ représentent, indépendamment l'un de l'autre,
(i) un atome d'hydrogène, d'halogène ou -NR¹¹R¹², ou
(ii) un radical alkyle en C₁-C₆ ou alcoxy en C₁-C₆, qui portent éventuellement chacun un ou plusieurs substituants, identiques ou différents, hydroxy, alcoxy en C₁-C₃, -NR⁸R⁹ ou hétérocyclyle, le cycle hétérocyclyle pouvant porter un ou plusieurs substituants, identiques ou différents, alkyle en C₁-C₃ ou -C(O)-R⁷,
R⁵ et R⁶ représentant, indépendamment l'un de l'autre, un atome d'hydrogène ou un radical alkyle en C₁-C₆ qui porte éventuellement un ou plusieurs substituants, identiques ou différents, hydroxy, alcoxy en C₁-C₃, -NR⁸R⁹ ou hétérocyclyle, le cycle hétérocyclyle pouvant porter un ou plusieurs substituants, identiques ou différents, alkyle en C₁-C₃ ou -C(O)-R⁷, ou
R⁵ et R⁶ pouvant également former ensemble, avec l'atome d'azote, un cycle à 5-7 chaînons, qui éventuellement contient en plus de l'atome d'azote un autre hétéroatome et qui porte éventuellement un ou plusieurs substituants, identiques ou différents, alkyle en C₁-C₆ et/ou -C(O)-R⁷, et
R⁷ représentant un radical alkyle en C₁-C₆, et
R⁸, R⁹, R¹⁰ représentant chacun indépendamment un atome d'hydrogène ou un radical alkyle en C₁-C₆, et
R¹¹ et R¹² représentant, indépendamment l'un de l'autre, un atome d'hydrogène ou un radical alkyle en C₁-C₆ qui porte éventuellement un ou plusieurs substituants, identiques ou différents, hydroxy, alcoxy en C₁-C₃, -NR⁸R⁹ ou hétérocyclyle, le cycle hétérocyclyle pouvant porter un ou plusieurs substituants, identiques ou différents, alkyle en C₁-C₃ ou -C(O)-R⁷,
ainsi que leurs sels, énantiomères et diastéréoisomères.

2. Composés de formule générale (I) présentant les éléments constitutifs A, B, C et D,
dans lesquels
les éléments constitutifs B et D se trouvent en position ortho l'un par rapport à l'autre et
Q₁ représente un cycle pyridinyle, pyridazinyle, pyrimidinyle, pyrazinyle ou triazinyle, et
R¹ et R² représentent indépendamment l'un de l'autre
(i) un atome d'hydrogène ou d'halogène, ou un groupe hydroxy, cyano, -CF₃, -NR⁵R⁶ ou
(ii) un radical alkyle en C₁-C₆, alcoxy en C₁-C₆, fluoroalkyle(C₁-C₆) ou un radical fluoroalcoxy(C₁-C₆) ou
(iii) -SO₂-R⁷, et
Q₂ représente un cycle phényle, thiényle, thiazolyle, furanyle, pyrrolyle, oxazolyle, imidazolyle, pyrazolyle, isoxazolyle, isothiazolyle, 1,2,3-triazolyle, pyridinyle, pyridazinyle, pyrimidinyle, pyrazinyle, 1,2,3,4-tétrahydro-isoquinolinyle, 1,2,3,4- tétrahydroquinolinyle, 2,3-dihydro-1H-indolyle, 2,3-dihydro-1H-iso-indolyle, 4,5,6,7-tétrahydro-thiéno[2,3-c]pyridinyle, 4,5,6,7-tétrahydro-thiéno[3,2-c]pyridinyle, 2,3-dihydro-benzofuranyle, benzo[1,3]dioxolyle, 2,3-dihydro-benzo[1,4]dioxinyle, 1,2,3,4-tétrahydro-quinoxalinyle ou un cycle 3,4-dihydro-2H-benzo[1,4]oxazinyle, et
R³ et R⁴ représentent, indépendamment l'un de l'autre,
(i) un atome d'hydrogène, d'halogène, -NR¹¹R¹² ou
(ii) un radical alkyle en C₁-C₆ ou alcoxy en C₁-C₆, éventuellement substitués chacun par morpholine ou -NR⁸R⁹,
R⁵ et R⁶ représentant, indépendamment l'un de l'autre, un atome d'hydrogène ou un radical alkyle en C₁-C₆ qui porte éventuellement un ou plusieurs substituants, identiques ou différents, hydroxy, -NR⁸R⁹, ou alcoxy en C₁-C₃, et
R⁷ représentant un radical alkyle en C₁-C₉, et
R⁸ et R⁹ représentent, indépendamment l'un de l'autre, un atome d'hydrogène ou un radical alkyle en C₁-C₄,
R¹¹ et R¹² représentant, indépendamment l'un de l'autre, un atome d'hydrogène ou un radical alkyle en C₁-C₆ qui porte éventuellement un ou plusieurs substituants, identiques ou différents, morpholine ou -N(CH₃)₂, -NH-CH₃ ou -NH-C₂H₅,
ainsi que leurs sels, énantiomères et diastéréoisomères.

3. Composés de formule générale (I) présentant les éléments constitutifs A, B, C et D selon la revendication 1 ou 2, dans lesquels
Q₁ représente un cycle pyridyle ou pyrazinyle,
ainsi que leurs sels, énantiomères et diastéréoisomères.

4. Composés de formule générale (I) présentant les éléments constitutifs A, B, C et D selon l'une quelconque des revendications 1 à 3, dans lesquels
R¹ et R² représentent indépendamment l'un de l'autre
(i) un atome d'hydrogène ou d'halogène, un groupe -NH₂, hydroxy, -CF₃, ou
(ii) un radical alcoxy en C₁-C₆, fluoroalkyle(C₁-C₆) ou un radical fluoroalcoxy(C₁-C₆),
ainsi que leurs sels, énantiomères et diastéréoisomères.

5. Composés de formule générale (I) présentant les éléments constitutifs A, B, C et D selon l'une quelconque des revendications 1 à 4, dans lesquels
Q₂ représente un cycle phényle, pyrazolyle, thiényle, pyridinyle ou 4,5,6,7-tétrahydrothiéno[2,3-c]pyridinyle,
ainsi que leurs sels, énantiomères et diastéréoisomères.

6. Composés de formule générale (I) présentant les éléments constitutifs A, B, C et D, selon l'une quelconque des revendications 1 à 5, dans lesquels
R³ et R⁴ représentent, indépendamment l'un de l'autre, un atome d'hydrogène, d'halogène ou un groupe alkyle en C₁-C₆,
ainsi que leurs sels, énantiomères et diastéréoisomères.

7. Composés de formule générale (I) présentant les éléments constitutifs A, B, C et D, dans lesquels
les éléments constitutifs B et D se trouvent en position ortho l'un par rapport à l'autre et
Q₁ représente un cycle pyridyle ou pyrazinyle, et
R¹ et R² représentent indépendamment l'un de l'autre
(i) un atome d'hydrogène ou d'halogène, un groupe -NH₂, hydroxy, -CF₃ ou
(ii) un radical alcoxy en C₁-C₆, fluoroalkyle(C₁-C₆) ou un radical fluoroalcoxy (C₁-C₆),
Q₂ représente un cycle phényle, pyrazolyle, thiényle, pyridinyle ou 4,5,6,7-tétrahydrothiéno[2,3-c]pyridinyle, et
R³ et R⁴ représentent, indépendamment l'un de l'autre, un atome d'hydrogène, d'halogène ou un groupe alkyle en C₁-C₆,
ainsi que leurs sels, énantiomères et diastéréoisomères.

8. Composés selon l'une quelconque des revendications 1 à 7, à savoir
N-[2-(aminocarbonyl)phényl]-2-(3-pyridinyl)-4-thiazolecarboxamide
N-[5-(aminocarbonyl)-1-méthyl-1H-pyrazol-4-yl]-2-(4-pyridinyl)-4-thiazolecarboxamide
N-[2-(aminocarbonyl)phényl)-2-(4-pyridinyl)-4-thiazolecarboxamide
N-(2-(aminocarbonyl)-4-méthylphényl]-2-(3-pyridinyl)-4-thiazolecarboxamide
N-[2-(aminocarbonyl)-5-méthylphényl]-2-(3-pyridinyl)-4-thiazolecarboxamide
N-(2-(aminocarbonyl)-4-chlorophényl]-2-(3-pyridinyl)-4-thiazolecarboxamide
3-[(2-pyridin-3-yl-thiazole-4-carbonyl)amino]pyridin-2-carboxamide
N-[2-(aminocarbonyl)-5-méthylphényl]-2-(4-pyridinyl)-4-thiazolecarboxamide
6-méthyl-2-[(2-pyridin-4-yl-thiazole-4-carbonyl)-amino]-4,5,6,7-tétrahydro-thiéno[2,3-c]pyridin-3-carboxamide
(3-carbamoyl-1-méthyl-1H-pyrazol-4-yl)-amide d'acide 2-pyridin-4-yl-thiazole-4-carboxylique
N-[2-(aminocarbonyl)-5-méthylphényl]-2-[4-(trifluorométhyl)-3-pyridinyl]-4-thiazolecarboxamide
N-[2-(aminocarbonyl)-5-méthylphényl]-2-[6-(2,2,2-trifluoroéthoxy)-3-pyridinyl]-4-thiazolecarboxamide
N-[2-(aminocarbonyl)-5-méthylphényl]-2-(6-méthoxy-3-pyridinyl)-4-thiazolecarboxamide
N-[3-(aminocarbonyl)-1-méthyl-1H-pyrazol-4-yl]-2-(pyrazin-2-yl)-4-thiazolecarboxamide
6-méthyl-2-[(2-pyrazin-2-yl-thiazole-4-carbonyl)-amino]-4,5,6,7-tétrahydro-thiéno[2,3-c]pyridin-3-carboxamide
N-[5-(aminocarbonyl)-1-méthyl-1H-pyrazol-4-yl]-2-(3-pyridinyl)-4-thiazolecarboxamide
N-[2-(aminocarbonyl)-5-(1,1-diméthyléthyl)-3-thiényl]-2-(3-pyridinyl)-4-thiazolecarboxamide
N-[2-(aminocarbonyl)-4-fluorophényl]-2-(3-pyridinyl)-4-thiazolecarboxamide
N-[2-(aminocarbonyl)-3-thiényl]-2-(3-pyridinyl)-4-thiazolecarboxamide
N-[3-(aminocarbonyl)-4-méthyl-2-thiényl]-2-(3-pyridinyl)-4-thiazolecarboxamide
3-[(2-pyridin-4-yl-thiazole-4-carbonyl)-amino]-pyridine-2-carboxamide
3-[(2-pyridin-4-yl-thiazol-4-carbonyl)-amino]-isonicotinamide
N-[2-(aminocarbonyl)phényl]-2-(6-méthoxy-3-pyridinyl)-4-thiazolecarboxamide
N-[2-(aminocarbonyl)phényl]-2-(2-méthoxy-3-pyridinyl)-4-thiazolecarboxamide
N-[2-(aminocarbonyl)phényl]-2-(5-chloro-3-pyridinyl)-4-thiazolecarboxamide
N-[2-(aminocarbonyl)-5-méthylphényl]-2-(6-aminopyridin-3-yl)-thiazole-4-carboxamide
N-[2-(aminocarbonyl)-5-méthylphényl)-2-(6-hydroxypyridin-3-yl)-thiazole-4-carboxamide

9. Procédé pour la préparation de composés selon l'une quelconque des revendications 1 à 8, **caractérisé par** les étapes
a) conversion de composés intermédiaires de formule (V) en composés intermédiaires de formule (IV) par mise en réaction avec du bromopyruvate d'éthyle par chauffage dans des solvants organiques, 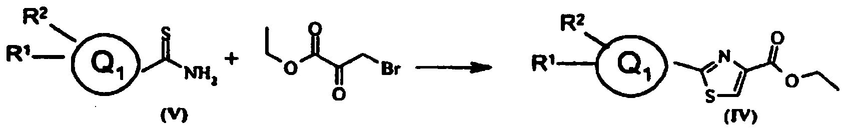
b) saponification de l'ester éthylique (IV) avec obtention de composés intermédiaires de formule (III) 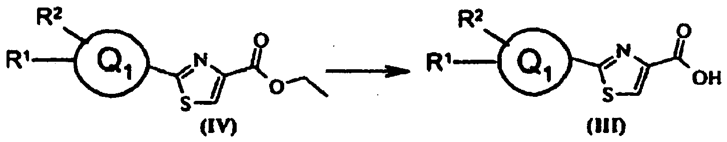
c) mise en réaction des composés intermédiaires de formule (III) avec des composés de formule (II) à l'aide d'un réactif de couplage d'amides 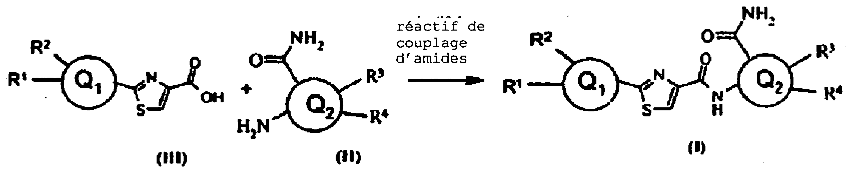 Q₁, Q₂, R¹, R², R³ et R⁴ ayant les significations indiquées dans la formule générale (I) selon les revendications 1 à 7.

10. Procédé pour la préparation de composés selon l'une quelconque des revendications 1 à 8, **caractérisé par** les étapes
a) mise en réaction d'acide 2-bromothiazole-4-carboxamide avec des composés intermédiaires de formule (II) à l'aide d'un réactif de couplage d'amides 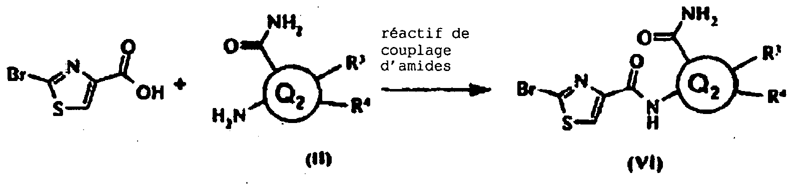
b) mise en réaction des composés intermédiaires de formule (VI) avec des acides boroniques ou des boronates de pinacol, dans le cadre d'une réaction de Suzuki-Miyaura, pour l'obtention de composés de formule (I) 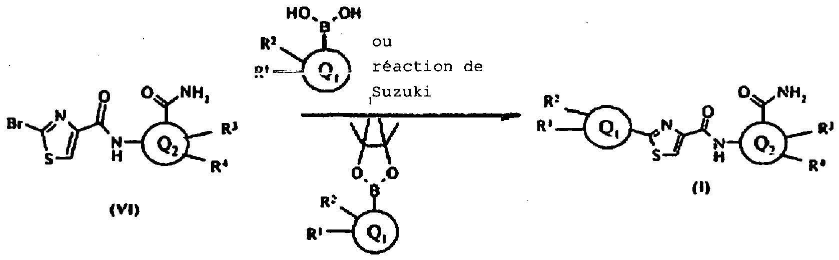 Q₁, Q₂, R¹, R², R³ et R⁴ ayant les significations indiquées dans la formule générale (I) selon les revendications 1 à 7.

11. Composés selon l'une quelconque des revendications 1 à 8, pour utilisation en tant que médicament.

12. Composés selon l'une quelconque des revendications 1 à 8, pour utilisation en tant que médicament contre des maladies qui s'accompagnent de processus inflammatoires, allergiques et/ou prolifératifs.

13. Composition pharmaceutique contenant un composé selon l'une quelconque des revendications 1 à 8.
